# EUROPEAN PATENT SPECIFICATION

(11) **EP 4 697 996 B1**
(45) Date of publication and mention of the grant of the patent: **16.09.2026**
(21) Application number: 24729560.3
(22) Date of filing: 03.05.2024
(51) Int. Cl.: A41D 1/00, A41D 1/04, A61H 1/00, A61F 5/02, A61F 5/01

(54) **UPPER BODY AND ARM COMPRESSION GARMENT SYSTEM**
OBERKÖRPER- UND ARMKOMPRESSIONSKLEIDUNGSSYSTEM
SYSTÈME DE VÊTEMENT DE COMPRESSION DU HAUT DE CORPS ET DE BRAS

(30) Priority: 05.05.2023 US 202363464464 P
(43) Date of publication of application: 25.02.2026
(73) Proprietor: Tactile Systems Technology, Inc., Minneapolis, MN 55416 (US)
(72) Inventor: STAAB, Ryan, Minneapolis, Minnesota 55416 (US); GAMBLE, Kristian D., Minneapolis, Minnesota 55416 (US); FARMER, Brian, Minneapolis, Minnesota 55416 (US); LINDRUD, Shad, Minneapolis, Minnesota 55416 (US); BECKER, Veronica H., Minneapolis, Minnesota 55416 (US)
(74) Representative: PGA S.p.A., Milano, Succursale di Lugano
(86) International application number: PCT/US2024/027829
(87) International publication number: WO 2024/233385

(56) References cited:
- CA-A1- 3 000 992
- US-A1- 2002 042 585
- US-A1- 2009 113 596
- US-B2- 6 860 862

## Description

This application claims the benefit of U.S. Provisional Application No. 63/464,464, filed May 5,.

The present disclosure relates generally to the use of compression garments and compression garment systems that are donned on a portion of the body of a user. Once donned, the compression garments may apply pressure to one or more regions of a portion of the body such as, for example, upper body regions, torso regions, upper arm regions, arm regions, etc.

Various types of compression garments may be used, for example, for treatment of lymphedema, edema, wound healing, etc. For example, garments may include inflatable chambers or cells (or other actuatable elements) to provide therapy to patients and may be positioned about any one or more body portions of a person or animal. Specifically, the garments may be positioned about body portions that exhibit swelling due to a build-up of lymph and that would benefit from compression therapy provided by the garments. For example, such chambers or cells may be inflatable to one or more different pressures in a variety of sequences to provide the therapy to the patient by moving lymph from one region to another. In other words, such compression garments may be placed around at least a portion of an individual's body for use in applying pressure to the body at an affected extremity. These compression garments may be donned (e.g., put on) and doffed (e.g., taken off) by patients themselves or with help from others. CA3000992 A1 describes compression garments designed for the head and torso.

### SUMMARY

The exemplary garments described herein may include an upper body garment that includes a torso portion and at least one upper arm portion (e.g., covering the shoulder and bicep regions). Further, the upper body garment may include a plurality of pressure applying regions that are controllable to apply pressure to a plurality of portions of the user wearing the garment. Specifically, the upper body garment provides coverage at the underarm region (e.g., the armpit) due to the intersection between the torso portion and the at least one upper arm portion. Therefore, the upper body garment may help to provide pressure to an underarm region of the user wearing the garment (e.g., the underarm region may be an area susceptible to a build-up of lymph).

Additionally, the upper body garment may include an underarm strap positionable at the underarm region (e.g., the armpit) of the user to assist in applying additional pressure to the underarm region. The underarm strap may include a plurality of pressure applying regions (e.g., one or more cells configured to receive fluid) that are controllable to apply pressure to the underarm region. The combination of the upper body garment and the underarm strap provide a focus of pressure applied at the underarm region of the user.

In one or more embodiments, the exemplary garments described herein may also include an arm garment that may be overlap with a portion of the upper body garment. The arm garment may include a plurality of arm pressure applying regions controllable to apply pressure to a plurality of portions of the arm. Therefore, the exemplary garments may be combined for a system in which pressure is applied to the body such that lymph is directed to move from a distal region of the arm to a proximal region of the arm and towards a core of the torso.

One exemplary compression garment system may include an upper body garment positionable proximate an upper body of a user and a controller operably coupled to the upper body garment. The upper body garment may include a torso garment portion, a left upper arm garment portion, and a right upper arm garment portion. The upper body garment may include a plurality of pressure applying regions controllable to apply pressure to a plurality of portions of the upper body. The plurality of pressure applying regions may include a plurality of torso pressure applying regions, at least one left upper arm pressure applying region, and at least one right upper arm pressure applying region. The controller may control pressure applied by the plurality of pressure applying regions to the upper body. The plurality of torso pressure applying regions may be configured to apply pressure to a plurality of portions of a torso, the at least one left upper arm pressure applying region may be configured to apply pressure to a portion of a left upper arm, and the at least one right upper arm pressure applying region may be configured to apply pressure to a portion of a right upper arm.

Another exemplary compression garment system may include an upper body garment positionable proximate an upper body of a user and a controller operably coupled to the upper body garment. The upper body garment may include a torso garment portion and at least one upper arm garment portion. The upper body garment may include a plurality of pressure applying regions controllable to apply pressure to a plurality of portions of the upper body. The upper body garment may also include at least one underarm strap positionable proximate an underarm region of the user between the torso garment portion and the at least one upper arm garment portion. The plurality of pressure applying regions may include at least one underarm pressure applying region. The controller may control pressure applied by the plurality of pressure applying regions to the upper body. The at least one underarm pressure applying region is configured to apply pressure to a portion of the underarm region.

Yet another exemplary compression garment system may include an arm garment positionable proximate an arm of a user and a controller operably coupled to the arm garment. The arm garment may extend between a distal end region and a proximal end region along a longitudinal axis. The arm garment may include a plurality of arm pressure applying regions controllable to apply pressure to a plurality of portions of the arm. The arm garment may include a hand strap attached to the arm garment proximate the distal end region. The hand strap may define an opening between an interior surface of the arm garment and the hand strap. The hand strap may be configured to be gripped by the user such that the distal end region of the arm garment maintains position relative to the arm and a remainder of the arm garment is positionable relative to the arm. The controller may control pressure applied by the plurality of arm pressure applying regions to the arm.

The above summary is not intended to describe each embodiment or every implementation of the present disclosure. A more complete understanding will become apparent and appreciated by referring to the following detailed description and claims taken in conjunction with the accompanying drawings.

### BRIEF DESCRIPTION OF THE DRAWINGS

FIG. 1A is a front view of an exemplary compression garment system including an upper body garment.
FIG. 1B is a front view of another exemplary compression garment system including an upper body garment.
FIG. 2 is a front view of the upper body garment of FIG. 1B in an open position showing an interior of the upper body garment.
FIG. 3 is an enlarged view of two straps and a slider of the upper body garment to assist in tightening the upper body garment on a user.
FIG. 4 is a rear view of the upper body garment of FIG. 1B.
FIG. 5 is a front view of an exemplary compression garment system including an arm garment.
FIG. 6A is a top view of the arm garment of FIG. 5 in an open position illustrating an interior surface of the arm garment.
FIG. 6B is a bottom view of the arm garment of FIG. 5 in an open position illustrating an exterior surface of the arm garment.
FIG. 7A is an enlarged view of the arm garment of FIG. 6A illustrating a user's hand gripping a hand strap of the arm garment in a first configuration.
FIG. 7B is an enlarged view of the arm garment of FIG. 6A illustrating a user's hand gripping a hand strap of the arm garment in a second configuration.
FIG. 8A is a front view of an exemplary compression garment system including an upper body garment and an arm garment attached thereto.
FIG. 8B is a front view of an exemplary compression garment system including an upper body garment and two arm garments attached thereto.
FIG. 9 is a cross-sectional view of one or more cells including actuatable elements (e.g., inflatable chambers or cells) of an exemplary compression garment that may be used with one of the exemplary garment portions.
FIG. 10A is a perspective view of the arm garment of FIG. 5 being attached to the upper body garment of FIG. 1B.
FIG. 10B is another perspective view of FIG. 10A, illustrating the arm garment attached to the upper body garment.
FIG. 11A is a front view of another exemplary compression garment system including an upper body garment.
FIG. 11B is a rear view of the upper body garment of FIG. 11A.
FIG. 12A is a plan view of an exterior of an exemplary upper body garment of the compression garment system of FIG. 11A.
FIG. 12B is a plan view of an exterior of an exemplary upper body garment of the compression garment system of FIG. 11A.
FIG. 13 is a front view of donning an arm garment up the arm of the user towards an upper body garment.
FIG. 14 is a front view of the arm garment attached to the upper body garment of FIG. 13.
FIG. 15 is another front view of the arm garment attached to the upper body garment of FIG. 13.
FIG. 16 is another front view of an exemplary upper body garment of the compression garment system of FIG. 11A.
FIG. 17 is another front view of an exemplary upper body garment of the compression garment system of FIG. 11A.
FIG. 18 is another front view of an exemplary upper body garment of the compression garment system of FIG. 11A, including left and right straps.
FIG. 19 is another front view of an exemplary upper body garment of the compression garment system of FIG. 11A, including using the left and right wraparound portions.
FIG. 20 is another front view of an exemplary upper body garment of the compression garment system of FIG. 11A, including using the underarm strap.
FIG. 21 is another front view of an exemplary upper body garment of the compression garment system of FIG. 11A, including using the lower straps.
FIG. 22 is another front view of an exemplary upper body garment having two arm garments.
FIG. 23 is a front view of an exemplary upper body garment and an unwrapped arm garment of the compression garment system of FIG. 11A.
FIG. 24 is another front view of an exemplary upper body garment of the compression garment system of FIG. 11A.
FIG. 25 is a sequence of closing a distal end of an exemplary arm garment.

### DETAILED DESCRIPTION OF EXEMPLARY EMBODIMENTS

In the following detailed description of illustrative embodiments, reference is made to the accompanying figures of the drawing, which form a part hereof, and in which are shown, by way of illustration, specific embodiments which may be practiced. It is to be understood that other embodiments may be utilized and structural changes may be made without departing from (e.g., still falling within) the scope of the disclosure presented hereby.

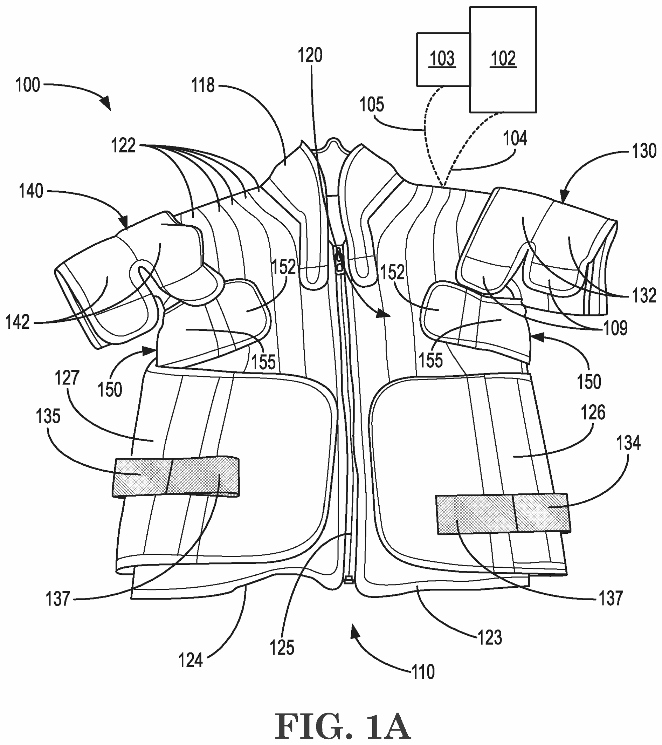

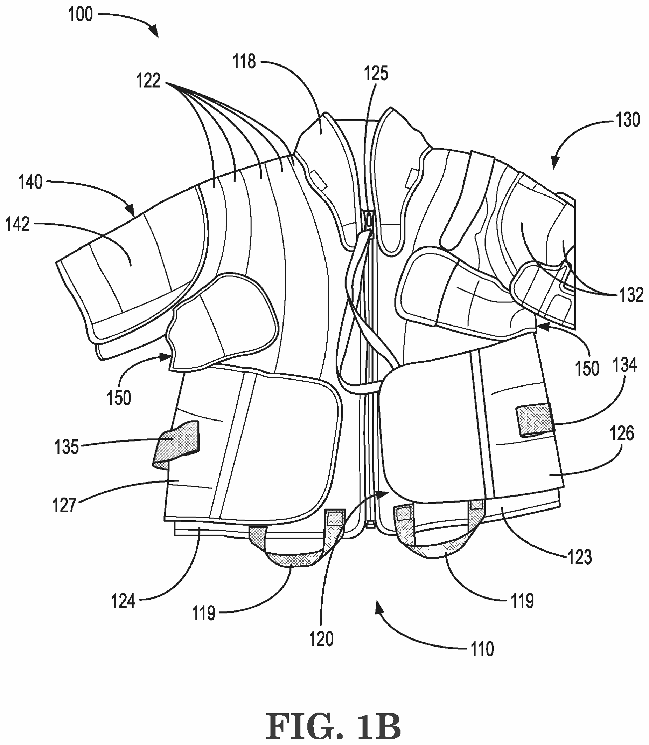

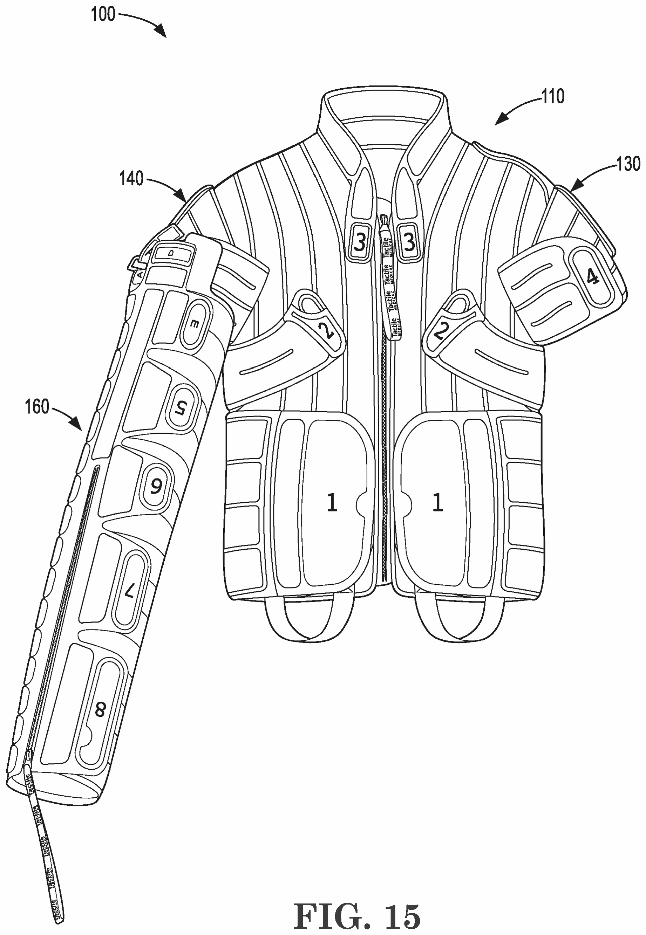

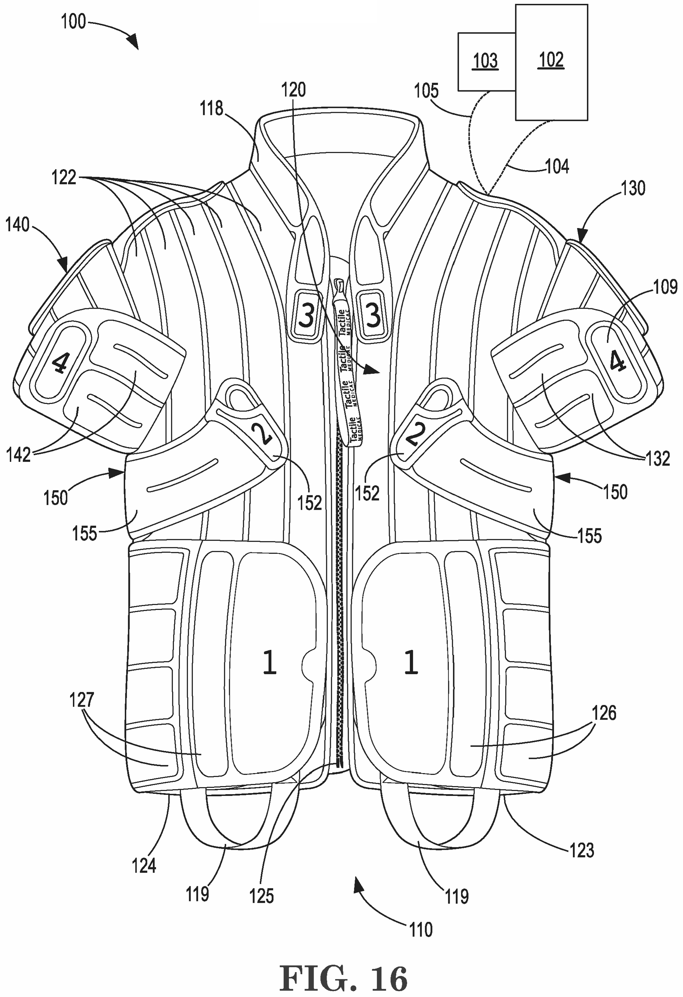

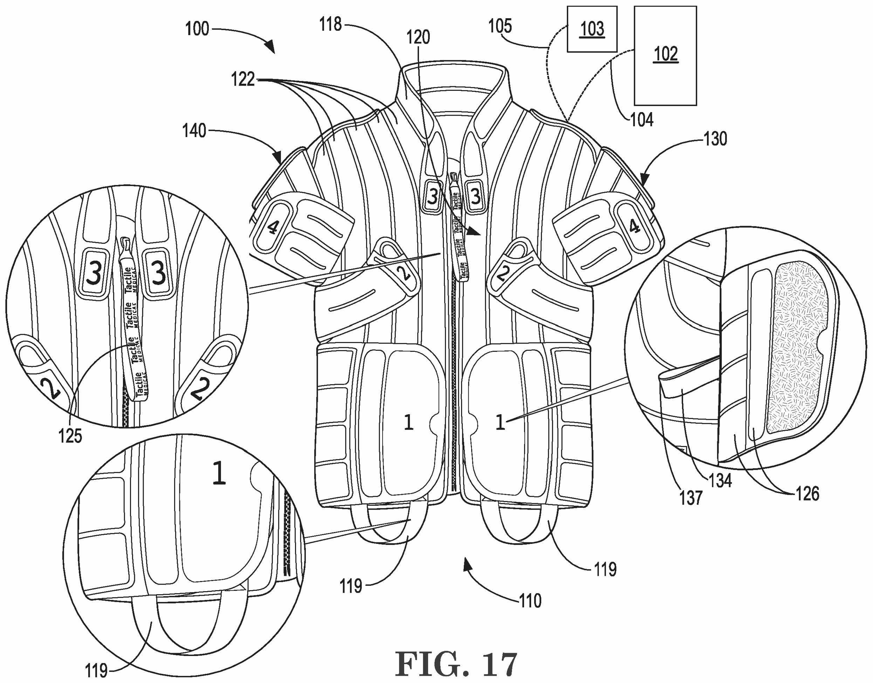

Exemplary garments, apparatus, systems, and structures shall be described with reference to FIGS. 1-25. It will be apparent to one skilled in the art that elements from one embodiment may be used in combination with elements of the other embodiments, and that the possible embodiments of such garments, apparatus, systems, and structures using combinations of features set forth herein is not limited to the specific embodiments shown in the Figures and/or described herein. Further, it will be recognized that the embodiments described herein may include many elements that are not necessarily shown to scale. Still further, it will be recognized that the size and shape of various elements herein may be modified but still fall within the scope of the present disclosure, although certain one or more shapes and/or sizes, or types of elements, may be advantageous over others.

The present disclosure relates generally to compression garments that include garment portions that are configured to be donned on portions of a body (e.g., person, animal, etc.) and configured to apply pressure to such portions of the body. The compression garments may include an upper body garment and an arm garment positionable on the corresponding body parts and controllable to apply pressure thereto.

Compression garment systems (e.g., such as compression garments described in U.S. Pat. No. 6,179,796 entitled "Lymphedema treatment system," U.S. Pat. No. 6,645,165 entitled "Lymphedema treatment system," U.S. Pat. No. 6,860,862 entitled "Lymphedema Treatment System," and U.S. Pat. No. 6,966,884 entitled "Lymphedema Treatment System," each of which may modify and be modified with features described herein) may be used for various reasons including therapy for people with lymphedema, animals requiring therapy, wound therapy, etc. As used herein, the term body refers to not only humans but any other animal species that may benefit from the concepts and features described herein. These compression garments may be placed around at least a portion of an individual's body and used to apply pressure to the body at an affected extremity (e.g., head, neck, arm, torso, a shoulder, leg, etc.). Some embodiments described herein may include a compression system having a garment configured to be positioned on (e.g., wrapped around, placed adjacent, located in proximity to, etc.) at least a portion of a body (e.g., human body, arm, torso, a shoulder, head, neck, etc.). The compression garments may be donned (e.g., put on) and doffed (e.g., taken off) by individuals themselves or with help from others. The garment may also include one or more chambers (e.g., cells, compartments, sealed volumes, bladders etc.) distributed (e.g., distributed throughout, distributed in concentric patterns "radiating" away from a central point or axis, along a length, etc.) of the garment configured to receive a fluid (e.g., air) to perform compression therapy.

The compression therapy provided by the compression garment systems may help to treat lymphedema. Lymphedema is a condition of localized fluid retention and tissue swelling that may be inherited, caused by cancer treatments, caused by parasitic infections, injury, surgery, etc. Compression garments described herein covering the torso, upper arms, and one or both arms may be used by an affected individual to provide a therapeutic benefit. Specifically, the compression garments may be configured to manipulate lymph nodes or vessels by applying pressure to move lymph toward more beneficial locations (e.g., toward drainage areas, away from affected regions, etc.). For example, compression therapy using the systems described herein may be performed around the torso and arm regions, or areas, to help treat lymphedema in the torso and arms by, e.g., moving lymph towards various lymph collection areas in the torso.

The compression garments described herein may be configured to apply pressure to the affected regions of the body to apply compression therapy. The compression garments may include various portions that each includes controllable pressure applying regions. Each controllable pressure applying region may be configured to apply pressure to a specific portion of the body (e.g., at a specific time during therapy). The controllable pressure applying regions may work in combination with one another to help provide therapy by applying a sequence of pressures on the body that moves lymph in a desired direction (e.g., from the hands down the arms and towards the torso, from one or more regions of the upper arm or torso to lymph collection regions of the torso, etc.). Such application of a sequence of pressures on the body that moves lymph (e.g., pressure being applied to one or more portions of the arm, shoulder, and torso at different times during a compression therapy period) may be referred to as applying dynamic pressure to the body. The sequence of pressures may be referred to as a pressure gradients, e.g., from a distal region to a proximal region. Additionally, in some embodiments, dynamic pressure may not be applied sequentially, and instead, be applied non-sequentially.

The controllable pressure applying regions of the compression garments may also apply static pressure to the body. For example, the compression garments may apply a constant pressure when a portion of the garment is positioned on, or about, the body over a therapy time period (e.g., static pressure over the therapy time period) or may apply a pressure that may be controlled to change over time during the therapy time period (e.g., dynamic pressure). In one or more embodiments, the dynamic pressure may be applied to the portion of the body through one or more chambers in the compression garment. The one or more chambers may be configured to receive fluid (e.g., air). Alternately, or in combination with one or more fluid receiving chambers, such pressures may be applied using one or more actuatable elements in the compression garment configured to apply pressure to the body (e.g., electrically controlled materials suitable to provide compression).

An exemplary compression garment system including a controller 102, a pump 103, and a garment (e.g., a compression garment) configured to be positioned around at least a portion of a body is shown in FIG. 1A. The controller, or control processor, 102 may be configured to control the pressure applied to one or more regions or portions of a user's body using the garment 100. For example, the controller 102 may control the pressure applied to each region or portion of the user's body using a plurality of individually controlled, independent pressure applying regions of the garment 100. Further, for example, pressure applying regions of the garment 100 may be controlled in groups or combinations. In one or more embodiments, the controller 102 may be configured to control the pressure applying regions in a variety of different sequences (e.g., applying pressure in a predetermined manner) that may be, e.g., suitable for carrying out lymphedema therapy.

Further, the controller 102 may control the pressure based on one or more pressures measured by one or more pressure sensors associated with the garment 100 (e.g., sensors provided in the garment 100 proximate the pressure applying regions) or with other portions of the system (e.g., sensors in communication with the pump, conduits, filling chambers or cells, etc.). One or more compression garments that may be modified with features (e.g., sensors) described herein may be similar to and include one or more features found in U.S. Pat. No. 6,860,862 entitled "Lymphedema Treatment System," U.S. Pat. No. 6,966,884 entitled "Lymphedema Treatment System," U.S. Pat. No. 6,179,796 entitled "Lymphedema treatment system," U.S. Pat. No. 6,645,165 entitled "Lymphedema treatment system," U.S. Patent No. 9,027,408 entitled "Elastomeric Particle Having An Electrically Conducting Surface, A Pressure Sensor Comprising Said Particles, A Method For Producing Said Sensor And A Sensor System Comprising Said Sensors," and U.S. Patent No. 7,947,003 entitled "Pressurized Medical Device," each of which is incorporated by reference in their entireties.

In one or more embodiments, the controller 102 (e.g., one or more processors employing one or more programs or routines carrying out one or more methods or processes and implemented with one or more types of memory) may be configured to control the system and/or one or more elements thereof (e.g., providing compression therapy by the one or more pressure applying regions, etc.). In one or more embodiments, the controller 102, or control processor, may be configured to control the compression system using wired and/or wireless technology.

The methods and/or logic and/or configurations described in this disclosure, including those attributed to the systems, or various constituent components, may be implemented, at least in part, in hardware, software, firmware, or any combination thereof. For example, various aspects of the techniques may be implemented within one or more processors, including one or more microprocessors, microcontrollers, DSPs, ASICs, FPGAs, or any other equivalent integrated or discrete logic circuitry, as well as any combinations of such components, or other devices. The term "processor" or "processing circuitry" may generally refer to any of the foregoing logic circuitry, alone or in combination with other logic circuitry, or any other equivalent circuitry.

Such hardware, software, and/or firmware may be implemented within the same device or within separate devices (e.g., within the system, outside of the system, or a combination of both) to support the various operations and functions described in this disclosure. In addition, any of the described components may be implemented together or separately as discrete but interoperable logic devices. Description of different features is intended to highlight different functional aspects and does not necessarily imply that such features must be realized by separate hardware or software components. Rather, functionality may be performed by separate hardware or software components, or integrated within common or separate hardware or software components.

When implemented in software, the functionality ascribed to the systems and methods described in this disclosure may be embodied as instructions and/or logic on a computer-readable medium such as RAM, ROM, NVRAM, EEPROM, FLASH memory, magnetic data storage media, optical data storage media, or the like. The instructions and/or logic may be executed by one or more processors to support one or more aspects of the functionality described in this disclosure.

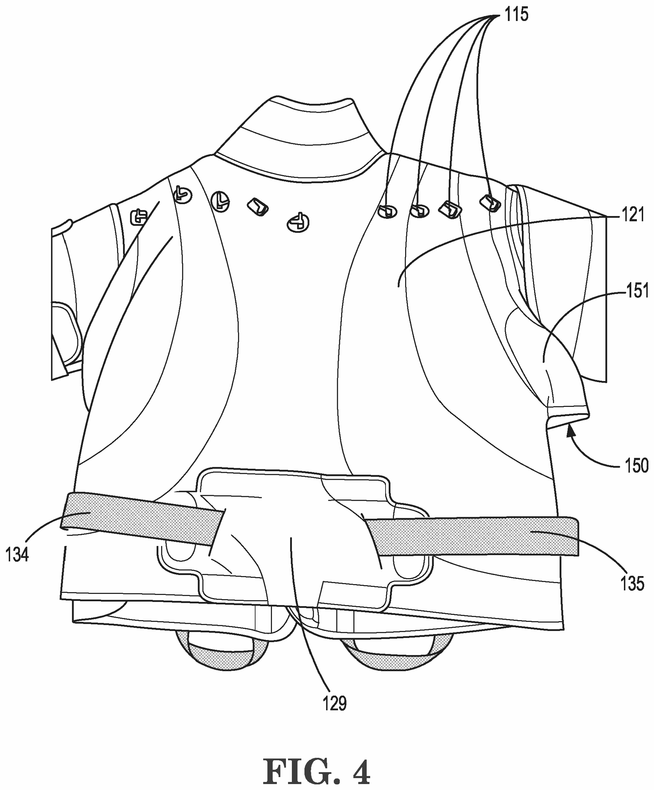

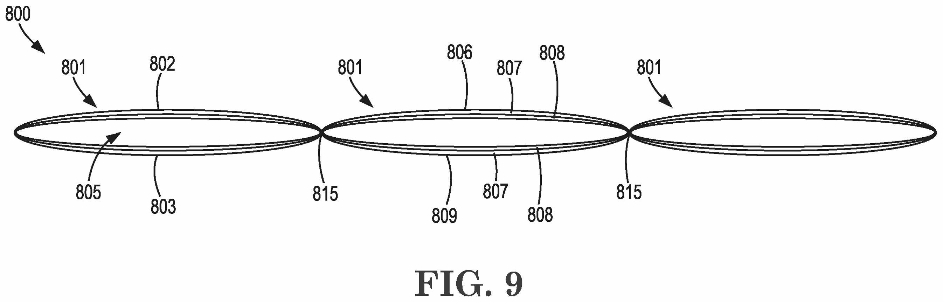

Further, the compression garment system may include a pump 103 that may be controlled by the controller 102 to provide a fluid to/from the one or more chambers (e.g., one or more chambers 801 as shown in FIG. 9) of each of pressure applying regions, e.g., a fluid such as a liquid or gas in the chambers, of the exemplary garment 100 so as to apply a compression therapy when the compression garment 100 is donned by a user. For example, the pump 103 may be connected to one or more of the plurality of chambers corresponding to the plurality of pressure applying regions by a plurality of lines or tubing 105 (e.g., including a tubing harness near the shoulder) so as to provide flow of fluid thereto or removal of fluid therefrom. Specifically, the upper body garment 110 may include ports 115 (e.g., as shown in FIG. 4) through which fluid may be provided to the one or more chambers.

Further, in one or more embodiments, as shown in FIG. 1A, the controller 102 may be connected to one or more components of the compression garment system via one or more electrical lines and/or wirelessly, as represented generally by dashed lines 104. For example, controller 102 may be connected to communicate and control the pressure applying regions (e.g., such as electrically actuatable pressure applying regions of the garment configured to apply pressure to the body) either with use of physical electrical connections and/or wirelessly.

The controllable pressure applying regions of the garment 100 under control of controller 102 may be described as allowing the system to provide compression therapy to an individual (e.g., a patient) wearing the garment 100 such that lymph flows throughout the user's body in desired directions, e.g., such as from a user's arm distally to proximally to the user's torso to regions or areas underneath, or proximate, the user's arm pit and axillary nodes. In other words, by controlling the pressure applying regions in a variety of different sequences (e.g., applying pressure in a predetermined manner), for example, lymph may flow generally from the hand of a user's arm down the arm towards the user's torso underneath the user's arm pit near or proximate the axillary nodes. Additionally, the exemplary system and garments described herein may be configured for use with a single arm or two arms, and further configured, to move lymph from one or both arms to one or both of the left and right axillary nodes.

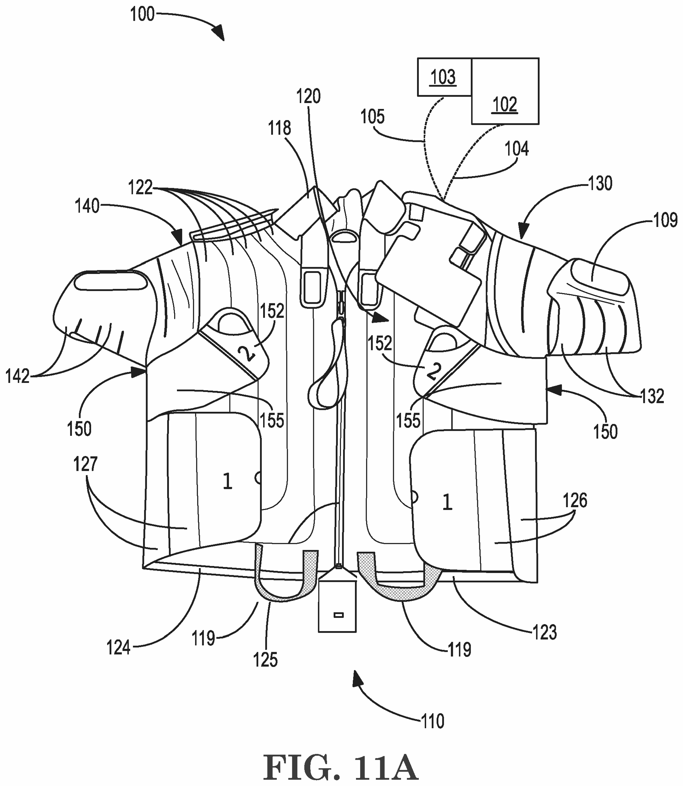

The exemplary garment 100 may include an upper body garment 110 positionable (e.g., configured to be positioned) proximate an upper body of a body (e.g., of a user). The upper body garment 110 may include a torso garment portion 120, a left upper arm garment portion 130, and a right upper arm garment portion 140 as shown in FIGS. 1A and 1B. A front view of additional embodiments of the upper body garment 110 is shown in FIGS. 11A, 16, and 17. The torso garment portion 120 may be positionable (e.g., configured to be positioned) proximate a torso of the body, the left upper arm garment portion 130 may be positionable (e.g., configured to be positioned) proximate a left upper arm of the body (e.g., the left shoulder and bicep region), and the right upper arm garment portion 140 may be positionable (e.g., configured to be positioned) proximate a right upper arm of the body (e.g., the right shoulder and bicep region).

The upper body garment 110 may define, or include, pressure applying regions located at regions of the upper body garment 110. The pressure applying regions may be controlled by the controller 102 and the pump 103 as described herein. In one or more embodiments, the upper body garment 110 may include an exterior material covering the pressure applying regions. Further, there may be multiple pressure applying regions that may be described as, e.g., a plurality of pressure applying regions. Each of the pressure applying regions may be controllable or configurable to apply pressure to a portion of the body (e.g., to portions of the upper body). For example, the pressure applying regions may include torso pressure applying regions 122 that may be controllable or configurable to apply pressure to a portion of the torso, at least one left upper arm pressure applying region 132 that may be controllable or configurable to apply pressure to a portion of the left upper arm, and at least one right upper arm pressure applying region 142 that may be controllable or configurable to apply pressure to a portion of the right upper arm. In other words, the controller 102 may be operably coupled to the upper body garment 110 to control pressure applied by the plurality of pressure applying regions to the upper body (e.g., and portions thereof).

The upper body garment 110 (e.g., the torso garment portion 120) may be described as configured to be positioned around both sides of the torso from the posterior of the torso to the anterior of the torso of the body. The upper body garment 110 (e.g., the left and right upper arm garment portions 130, 140) may also be described as configured to be positioned around one or both upper arms between the shoulder and bicep region. The left and right upper arm garment portions 130, 140 may extend from the torso garment portion 120 to form a continuous upper body garment 110. Although, in one or more embodiments, the pressure applying regions 122 of the torso garment portion 120 may be isolated or separate from the pressure applying regions 132, 142 of the left or right upper arm garment portions 130, 140.

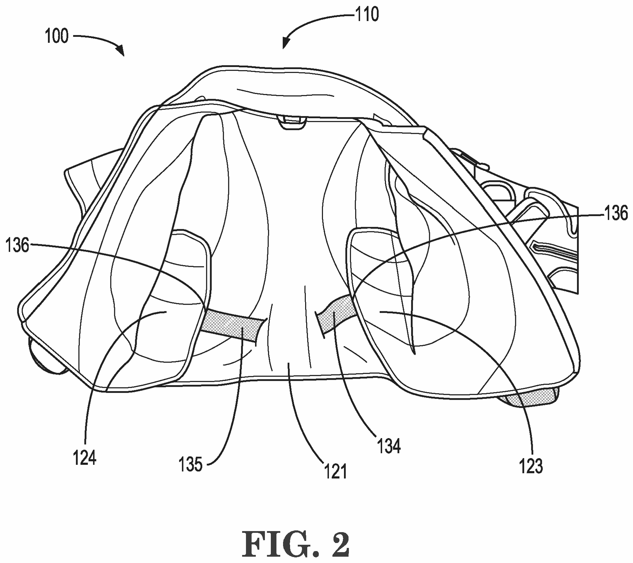

Furthermore, the torso garment portion 120 may include a posterior torso portion 121 (e.g., see FIG. 2), a left torso portion 123, and a right torso portion 124. The posterior torso portion 121 may be positionable proximate a posterior of the torso of the body, the left torso portion 123 may extend from the posterior torso portion 121 and be positionable to the anterior of the torso, and the right torso portion 124 may extend from the posterior torso portion 121 and be positionable to the anterior of the torso. The left upper arm garment portion 130 may extend from the posterior torso portion 121 and the left torso portion 123, and positionable to the left arm such that the left arm may extend outward through the left upper arm garment portion 130. The right upper arm garment portion 140 may extend from the posterior torso portion 121 and the right torso portion 124, and positionable to the right arm such that the right arm may extend outward through the right upper arm garment portion 140.

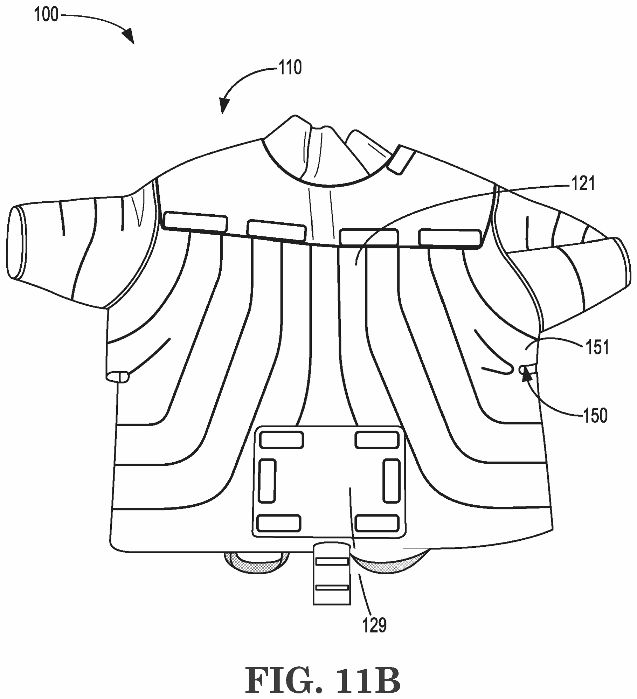

The left and right upper arm garment portions 130, 140 may be configured to be wrapped around the left and right upper arms of the body. For example, the left and right upper arm garment portions 130, 140 may be defined by a single garment portion that wraps around the left and right upper arm, respectively. After the arms are positioned relative to the upper arm garment portions 130, 140, the upper arm garment portions 130, 140 may be wrapped around the respective arm to form a tight or snug fit therewith. The upper arm garment portions 130, 140 may be wrapped in either direction (e.g., top-down as shown in FIGS. 1-10 or bottom-up as shown in FIGS. 11-25).The left and right upper arm garment portions 130, 140 may include attachment tabs 109 to maintain the upper arm garment portions 130, 140 wrapped around the upper arms of the body. For example, the attachment tabs 109 may assist in preventing the left and right upper arm garment portions 130, 140 from moving away from the surface of the upper arms of the body such that pressure may be effectively applied to the upper arms. Specifically, the attachment tabs 109 and the exterior surface of the upper arm garment portions 130, 140 may use hook-and-loop fasteners to removably couple to one another. Therefore, the user may insert each arm through the wrapped upper arm garment portions 130, 140 to don the garment for each subsequent use.

In one or more embodiments, the upper body garment 110 may also include at least one underarm strap 150 positionable proximate an underarm region of the body (e.g., as shown in FIGS. 1A and 1B). For example, the at least one underarm strap 150 may be positioned between the torso garment portion 120 and at least one of the upper arm garment portions 130, 140. Specifically, the at least one underarm strap 150 may include a left underarm strap positionable between the left upper arm garment portion and the torso garment portion, and a right underarm strap positionable between the right upper arm garment portion and the torso garment portion.

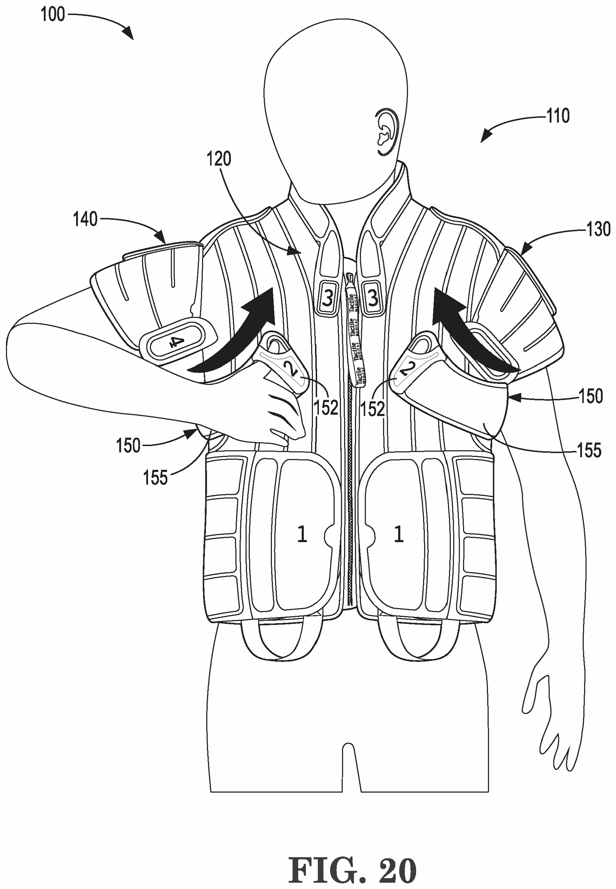

The at least one underarm strap 150 may extend from a first end region 151 (e.g., see FIG. 4) to a second end region 152. Further, the at least one underarm strap 150 may be configured to extend between a posterior of the upper body garment 110 and an anterior of the upper body garment 110 through the underarm region of the user. For example, the first end region 151 of the underarm strap 150 may be connected to the posterior of the upper body garment 110 and the second end region 152 of the underarm strap 150 may be connected to the anterior of the upper body garment 110. In one or more embodiments, the first end region 151 of the underarm strap 150 may be integrated with the posterior torso portion 121 (e.g., as shown in FIG. 4). Further, one or both of the first and second end regions 151, 152 may be removably couplable to the upper body garment 110. For example, the first end region 151 of the underarm strap 150 may be fixedly coupled (or integrated with the posterior torso portion 121) to the posterior of the upper body garment 110 and the second end region 152 of the underarm strap 150 may be removably couplable to the anterior of the upper body garment 110. Therefore, the second end region 152 of the underarm strap 150 may be positionable relative to the upper body garment 110 to securely position the underarm strap 150 at the desired location (e.g., to apply pressure to the underarm region of the body). In one or more embodiments, the underarm strap 150 may include a pocket or a portion (e.g., proximate an end of the underarm strap 150) for the user to grab (e.g., using one or more fingers and/or thumb) and pull/tighten the underarm strap 150 (e.g., as shown in FIG. 20).

The at least one underarm strap 150 may also include pressure applying regions 155. In other words, the plurality of pressure applying regions may include at least one underarm pressure applying region 155. As such, the at least one underarm strap 150 may apply pressure to the underarm region of the body statically by positioning (e.g., and tightening) the underarm strap 150 to extend through the underarm region. Further, the at least one underarm pressure applying region 155 may be configured or controllable to apply pressure (e.g., dynamically) to a portion of the underarm region. For example, the at least one under arm pressure applying region 155 may include one or more cells configured to receive a fluid (e.g., to apply a compression therapy as described herein).

The right and left torso portions 123, 124 may be coupled (e.g., removably couplable) to each other after donning the upper body garment 110 on the torso of the body to attach (e.g., secure) the upper body garment 110 to the body (e.g., attached proximate the anterior of the torso). The right torso portion 124 may be coupled to the left torso portion 123 in any suitable manner. For example, the left and/or right torso portions 123, 124 may include fastening apparatus to, e.g., fasten or couple a region of the right torso portion 124 to a portion of the left torso portion 123. Although the right torso portion 124 is coupled to the left torso portion 123 using a zipper 125 as shown, any fastening apparatus may be used such as, e.g., hook and loop fasteners, draw strings, buttons, etc.

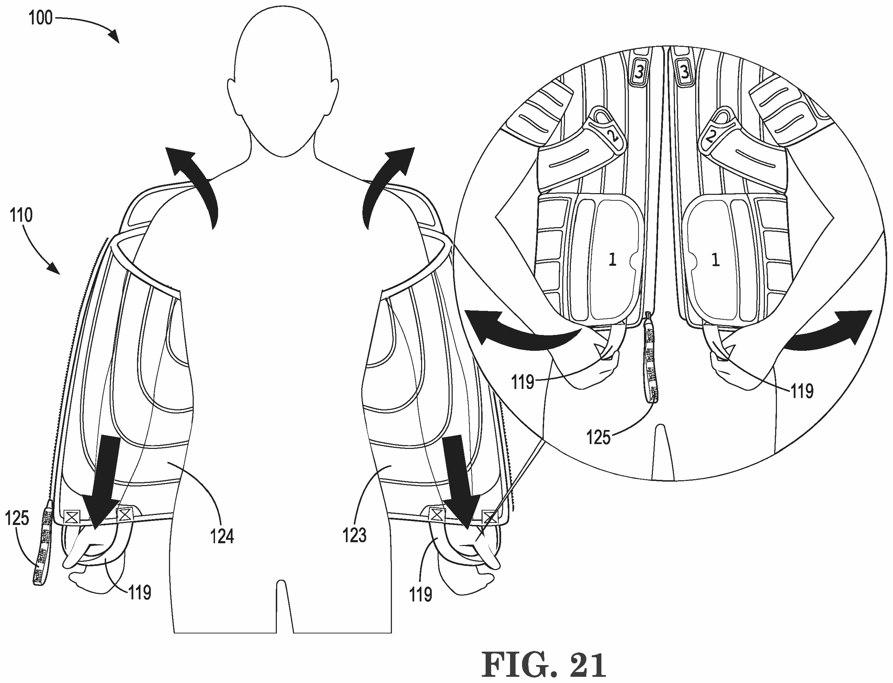

In one or more embodiments, the upper body garment 110 may also include lower straps 119 proximate the bottom edge of each of the left and right torso portions 123, 124 (e.g., as shown in FIG. 1B). The lower straps 119 may assist the user in adjusting the upper body garment 110 into position when donning. Further, in one more embodiments, the lower straps 119 may be used to help with doffing or taking off the upper body garment 110 (e.g., as shown in FIG. 21).

Additionally, the torso garment portion 120 may further include a left wraparound portion 126 and a right wraparound portion 127, which may be configured to further couple, or secure, the torso garment portion 120 about the torso of the body. More specifically, it may be described that the left and right wraparound portions 126, 127 may be configured to don, or wrap, and "tighten" the torso garment portion 120 about the torso of the body. For example, left wraparound portion 126 may extend from the posterior torso portion 121 to extend around the left side of the torso to the anterior of the torso and the right wraparound portion 127 may extend from the posterior torso portion 121 to extend around the right side of the torso to the anterior of the torso. Further, the left wraparound portion 126 may be removably couplable to at least the left torso portion 123 to tighten the torso garment portion 120 about the torso and the right wraparound portion 127 may be removably couplable to at least the right torso portion 124 to tighten the torso garment portion 120 about the torso. For example, the left wraparound portion 126 may be configured to overlap the left torso portion 123 (e.g., proximate the anterior portion of the torso) and the right wraparound portion 127 may be configured to overlap the right torso portion 124 (e.g., proximate the anterior portion of the torso) to, e.g., further don and/or tighten and/or secure the upper body garment 110 to the body. Specifically, the left and right wraparound portions 126, 127 may tighten the upper body garment 110 around the torso by moving the posterior torso portion 121 closer to the left and right torso portions 123, 124.

Furthermore, in one or more embodiments and/or depending on the size of the body with respect to the size of the upper body garment 110, the left wraparound portion 126 may be configured to overlap the midline of the torso and over to the right torso portion 124, and likewise, the right wraparound portion 127 may be configured to overlap the midline of the torso and over to the left torso portion 123. The left and right wraparound portions 126, 127 may be removably attached to the left and right torso portions 123, 124, respectively, in any suitable way. For example, the left and right wraparound portions 126, 127 may be removably attached to the left and right torso portions 123, 124 via hook and loop fasteners, buttons, etc.

In one or more embodiments, the upper body garment 110 may also include a neck garment portion 118 configured to apply pressure to the neck. The neck garment portion 118 may be coupled to the torso garment portion 120. In one or more embodiments, the neck garment portion 118 may include neck pressure applying regions controllable and configured to apply pressure to a portion of the neck.

The upper body garment 110 may include any suitable number of pressure applying regions to control the pressure applied to one or more portions of the body when the upper body garment 110 is positioned on the body. In other words, any number of pressure applying regions may be implemented or utilized to control (e.g., using controller 102 as shown in FIG. 1A) the flow of lymph through the body. For example, in one or more embodiments, the upper body garment 110 may include eight pressure applying regions on each side of the upper body garment 110 (e.g., on either side of a central axis of the body). Specifically, the torso garment portion 120 may include five torso pressure applying regions 122 on each side of the upper body garment 110 and each of the left and right upper arm garment portions 130, 140 may include two left/right upper arm pressure applying regions 132, 142. In other embodiments, it may be described that the left upper arm garment portion 130 may include at least one left upper arm pressure applying region 132 and the right upper arm garment portion 140 may include at least one right upper arm pressure applying region 142. In one or more embodiments, the chambers of the pressure applying regions of the upper body garment 110 may be in fluid communication with the corresponding chamber on the other side of the upper body garment 110. For example, the complementary chambers on each side of the upper body garment 110 (e.g., the outer most chamber of the torso garment portion 120 on the left and right sides, the underarm chamber on the left and right sides, etc.) may inflate and deflate together to apply compression therapy.

In one or more embodiments, each of the pressure applying regions may be configured in any suitable manner such that the regions may be controlled to apply pressure to a portion of the body to move lymph as desired. For example, the pressure applying regions may include fluid chambers or cells, pneumatic pressure applying regions, actuatable elements applying pressure to regions, hydraulic pressure applying regions, etc. Specifically, the pressure applying regions may include one or more chambers configured to receive fluid (e.g., air). Therefore, in one or more embodiments, each of the two left upper arm pressure applying regions 132 may include a cell (e.g., two cells total) configured to receive a fluid and each of the two right upper arm pressure applying regions 142 may include a cell (e.g., two cells total) configured to receive a fluid.

For example, in one or more embodiments, the pressure applying regions may be configured to apply pressure to a portion of the torso using the one or more chambers or cells through the control of fluid provided thereto, e.g., liquid flow, air flow, etc. In other words, the pressure applying regions may include one or more chambers or cells configured to receive a fluid. For example, the upper body garment 110 may include one or more garment ports 115 (e.g., as shown in FIG. 4) through which fluid may be provided to the one or more chambers (e.g., such as with use of pump 103 shown in FIG. 1A, under control of controller 102 with use of a sensor feedback system).

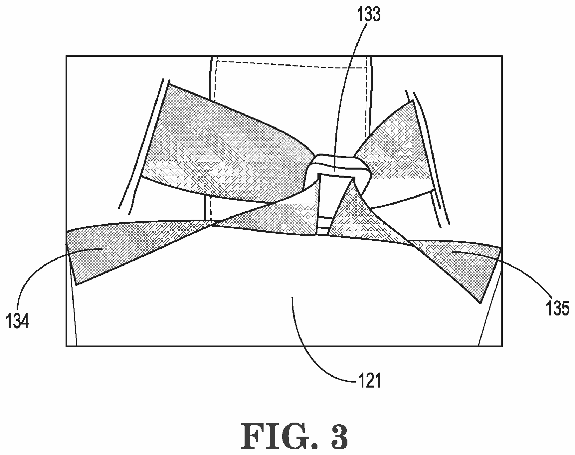

The torso garment portion 120 may further include a left strap 134 and a right strap 135 configured to adjust the torso garment portion 120 to properly fit around the circumference of the torso of the body (e.g., as shown in FIG. 1A). For example, the left strap 134 may be attached to a portion of the left torso portion 123 and extend inwards towards a central portion of the posterior torso portion 121 as shown in FIG. 2. Specifically, the left strap 134 may be attached or coupled to a rear edge portion of the left torso portion 123 as shown in FIG. 2. In one or more embodiments, the left strap 134 may extend through the posterior torso portion 121 (e.g., through a slit or opening) to a back exterior of the upper body garment 110 as shown in FIG. 3. The left strap 134 may thereafter extend outwards around the left torso portion 123 as shown in FIG. 1A. Likewise, the right strap 135 may be attached to a portion (e.g., a rear edge portion) of the right torso portion 124 and extends inwards towards a central portion of the posterior torso portion 121 as shown in FIG. 2. Thereafter, the right strap 135 may extend outwards around the right torso portion 124 as shown in FIG. 1A. Specifically, the left and right straps 134, 135 may be used to tighten the upper body garment 110 after the upper body garment 110 is donned on the body.

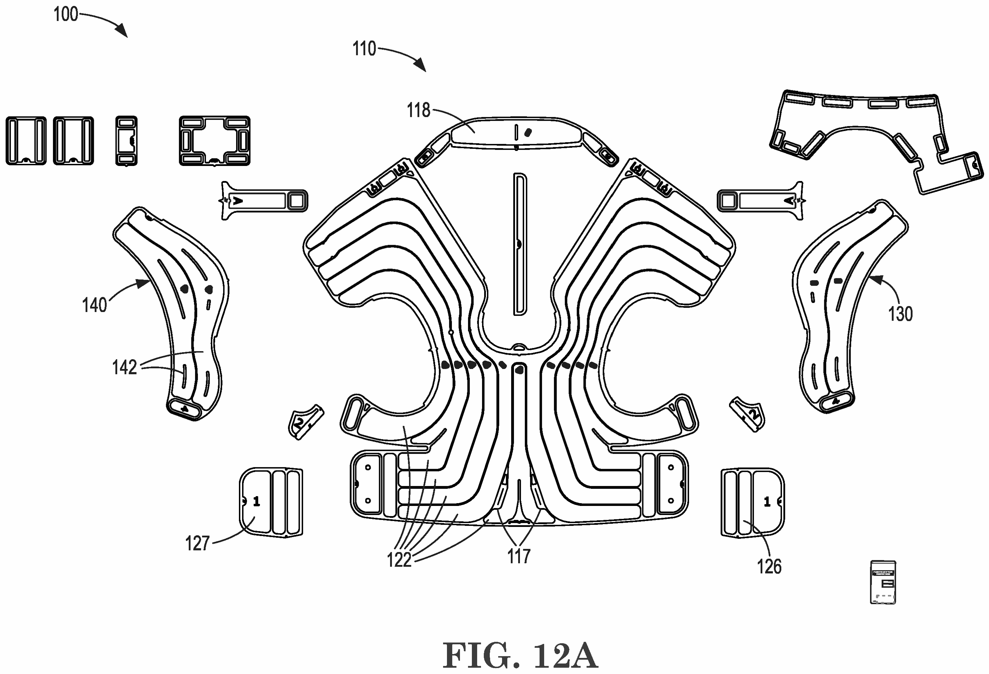

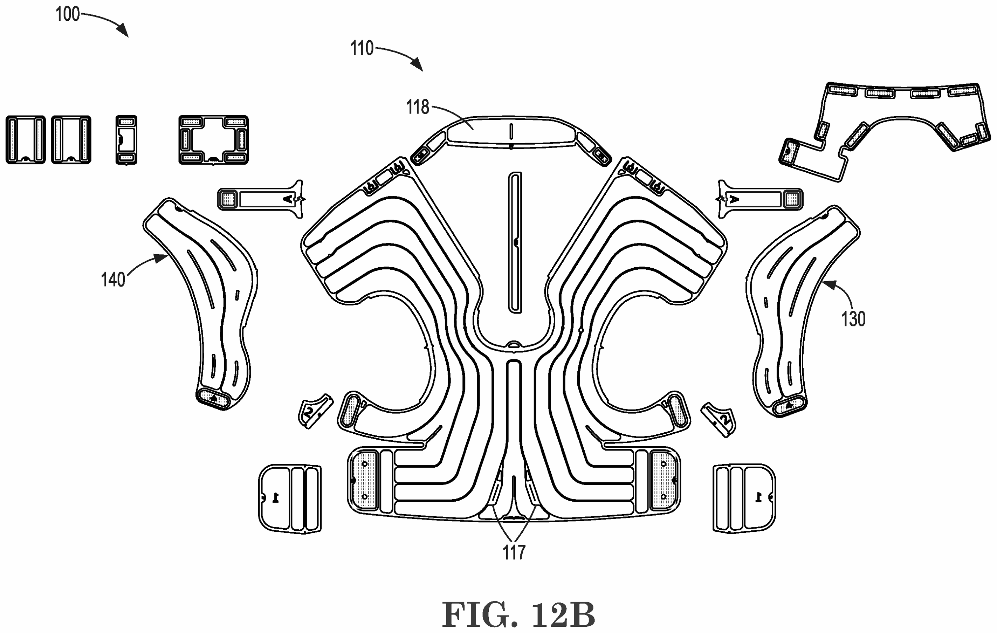

As shown in FIG. 3, the left and right straps 134, 135 may be movably connected to one another via a slider 133 defining two slots 117 (FIGS. 12A and 12B). For example, each of the left and right straps 134, 135 extend though the slider 133 to change direction from extending inwards towards the center to extending outwards and around the upper body garment 110. Therefore, because each of the left and right straps 134, 135 are attached to a rear edge portion of the left and right torso portions 123, 124, respectively, when the left and right straps 134, 135 are pulled around the torso, the motion is translated through the slider 133 to pull the rear edge portions of the left and right torso portions 123, 124 towards the center back to, e.g., tighten the upper body garment 110.

Additionally, the left strap 134 is removably couplable to the left torso portion 123 proximate the anterior of the torso and the right strap 135 is removably couplable to the right torso portion 124 proximate the anterior of the torso. For example, one or both of the left and right straps 134, 135 may be pulled around the torso (e.g., to tighten the upper body garment 110) and removably attached proximate the anterior to maintain a tight or snug fit of the upper body garment 110. Specifically, each of the left and right straps 134, 135 may extend from a proximal end 136 attached to the left and right torso portions 123, 124, respectively (e.g., as shown in FIG. 2), and a distal end 137 configured to be removably couplable to the left and right torso portions 123, 124, respectively (e.g., as shown in FIG. 1A). To facilitate or provide the coupling between the left strap 134 and the left torso portion 123 (or the right strap 135 and the right torso portion 124), each may include corresponding components of hook-and-loop fasteners configured to be coupled to one another. In one or more embodiments, the distal ends 137 of the left and right straps 134, 135 may include a loop that is configured to be manipulated or handled by a hand of the user (e.g., to more easily control the strap). Furthermore, in one or more embodiments, the torso garment portion 120 may also include a cover 129 (e.g., as shown in FIG. 4) attached to an exterior surface of the posterior torso portion 121 over the left and right straps 134, 135 proximate the central portion of the posterior torso portion 121 (e.g., over the location of the slider 133 through which the left and right straps 134, 135 extend). A rear view of another embodiment of the upper body garment 110 is shown in FIG. 11B.

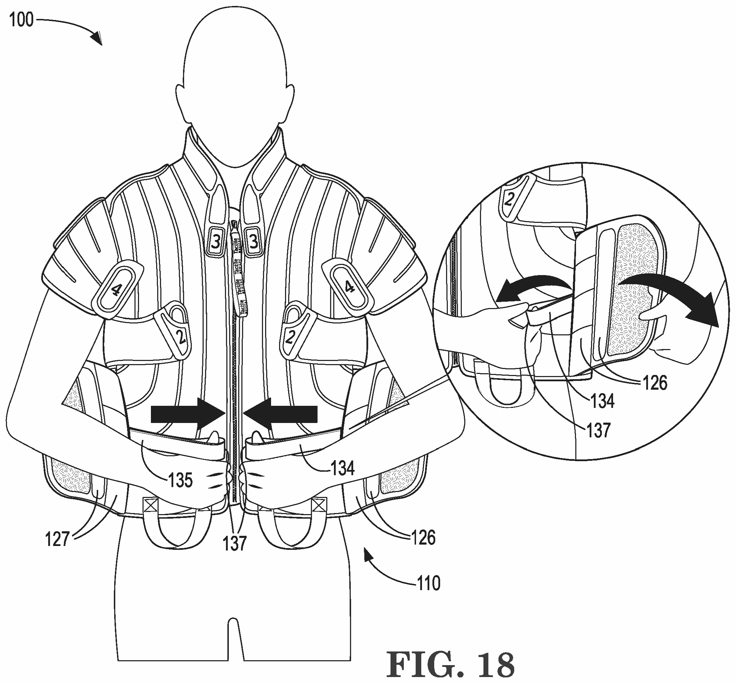

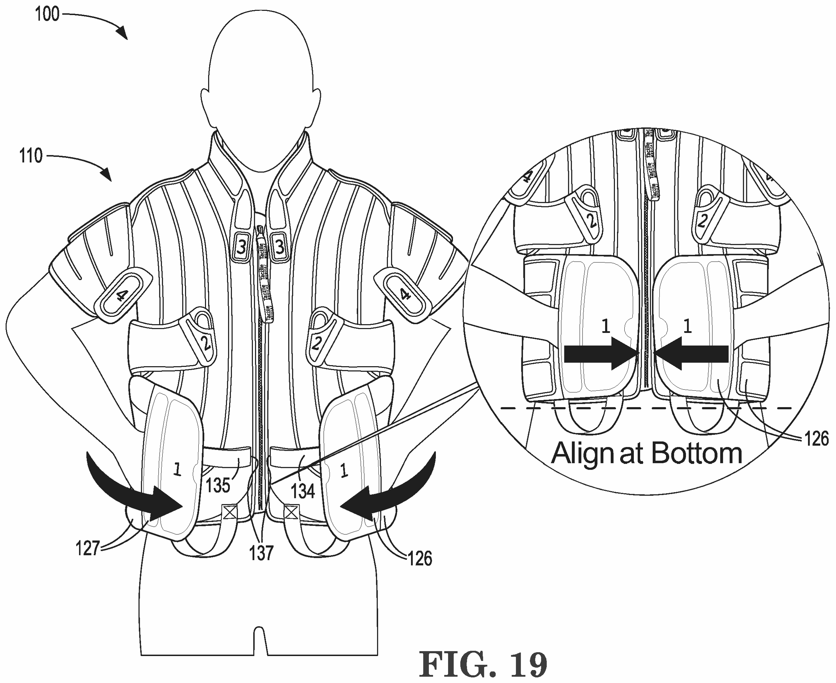

In one or more embodiments, the left and right straps 134, 135 (e.g., the distal ends 137 thereof) may be positioned underneath the left and right wraparound portions 126, 127 as shown in FIGS. 18 and 19. In other words, the left and right wraparound portions 126, 127 may cover the left and right straps 134, 135, respectively, to hide the left and right straps 134, 135 from view. As such, the user may adjust the tightness of the garment using left and right straps 134, 135 and then position the left and right wraparound portions 126, 127 over the left and right straps 134, 135 as shown in FIGS. 18 and 19.

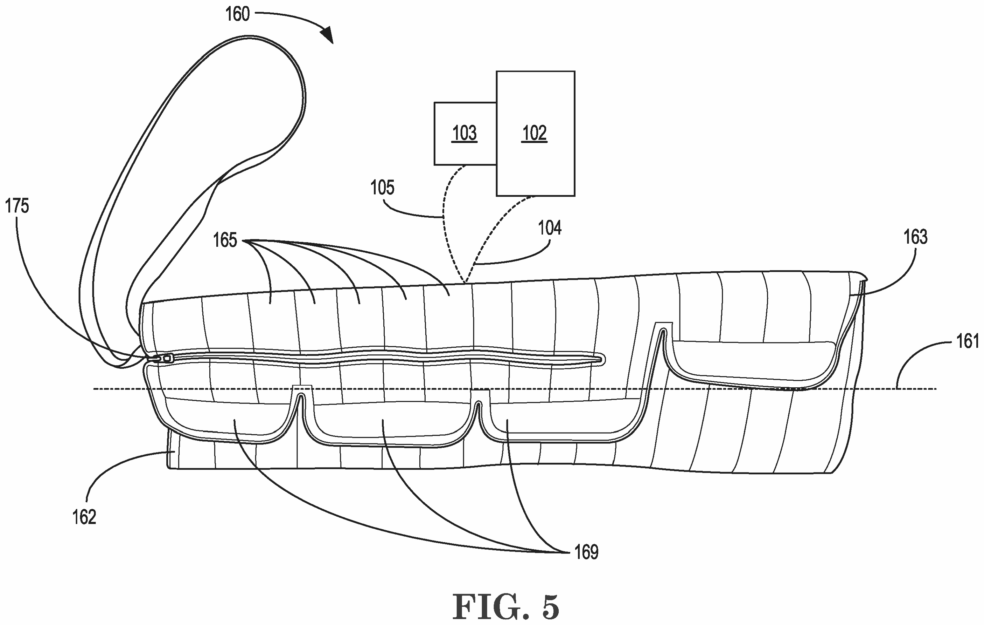

The compression garment system described herein may also include an arm garment 160, e.g., as shown in FIG. 5. The arm garment 160 may be configured to receive at least a portion of a user's arm (e.g., left or right arm) to provide compression therapy to the arm. In other words, the arm garment 160 may be positionable proximate the arm of the user. The arm garment 160 may extend between a distal end region 162 and a proximal end region 163 along a longitudinal axis 161. The arm of the user may be positioned such that the distal end region 162 of the arm garment 160 is proximate a hand of the arm and the proximal end region 163 of the arm garment 160 is proximate the upper arm or shoulder of the arm.

The arm garment 160 may include arm pressure applying regions 165 controllable to apply pressure to portions of the arm. For example, the arm pressure applying regions 165 may be similar to the pressure applying regions 165 described herein in connection with the upper body garment 160. In other words, the arm pressure applying regions 165 may be controlled by a controller 102 and a pump 103 as described herein. Specifically, a controller 102 may be operably coupled to the arm garment 160 and configured to control the arm pressure applying regions 165. Further, the controller 102 may be configured to control the arm pressure applying regions 165 in a variety of different sequences (e.g., applying pressure in a predetermined manner) that may be, e.g., suitable for carrying out lymphedema therapy.

Also, similar to as described above in connection with the upper body garment 110, a pump 103 may controlled by the controller 102 and operably coupled to the arm garment 160 to provide fluid to/from one or more chambers of the pressure applying regions to apply a compression therapy when the arm garment 160 is donned by a user. Further, the pump 103 may be connected to the chambers or cells corresponding to the arm pressure applying regions 165 by a plurality of lines or tubing 105 so as to provide flow of fluid thereto or removal of fluid therefrom. In one or more embodiments, the plurality of lines or tubing 105 associated with the arm garment 160 may include a tubing harness operably coupled to the arm garment 160 at an elbow region of the arm garment 160 (e.g., as shown in FIG. 5).

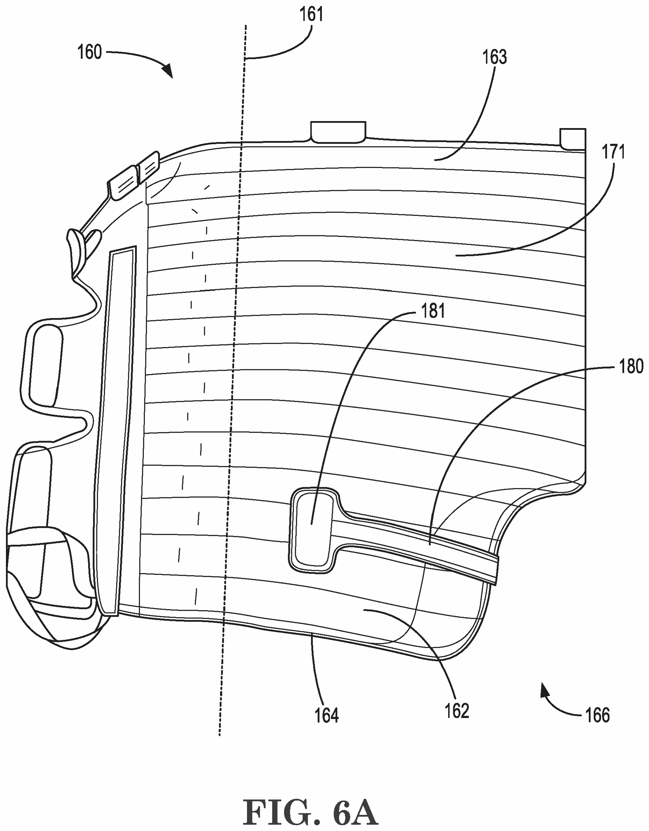

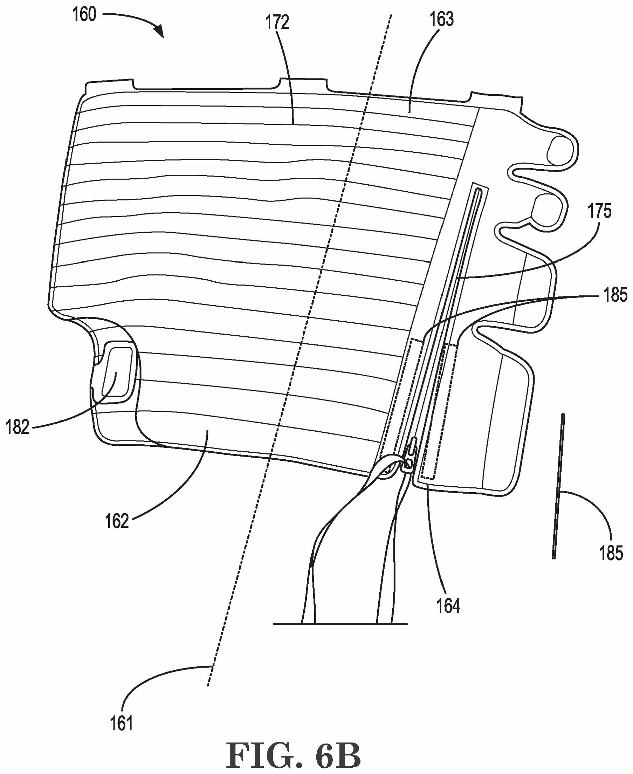

FIG. 6A illustrates an interior surface 171 of the arm garment 160 that is positionable against the arm of the user donning the arm garment. FIG. 6B illustrates an exterior surface 172 of the arm garment 160 that is positioned outwardly when the user is donning the arm garment 160. For example, a user may position an arm on the interior surface 171 of the arm garment 160 and wrap the arm garment 160 around the arm. Thereafter, attachment tabs 169 of the arm garment 160 may be utilized to removably couple the arm garment 160 to itself to maintain the arm garment 160 wrapped around the arm of the user. For example, the attachment tabs 169 may assist in preventing the arm garment 160 from moving away from the surface of the arm of the body such that pressure may be effectively applied to the arm. Specifically, the attachment tabs 169 and the exterior surface 172 may use hook-and-loop fasteners to removably couple to one another.

In one or more embodiments, the user may initially wrap the arm garment 160 around the arm to appropriately size and secure the arm garment 160. In subsequent uses, the user may insert the arm through an end opening of the arm garment 160 (e.g., at the proximal end region 163) to don the arm garment 160. In one or more embodiments, the arm garment 160 may include a zipper 175 to assist in donning a wrapped arm garment 160, as will be described further herein.

Additionally, in one or more embodiments, the arm garment may include a cutout section 166 on one side of the distal end region 162. The cutout section 166 may help to limit the amount of material present proximate the hand when the arm garment 160 is wrapped or donned on the arm.

Further, as shown in FIG. 6A, the arm garment 160 may include a hand strap 180 attached to the arm garment 160 proximate the distal end region 162. The hand strap 180 may be configured to be gripped or handled by the user (e.g., using a hand of the arm within the arm garment 160) to assist with donning the arm garment 160. For example, the hand strap 180 may extend between a first end 181 attached to the arm garment 160 and a second end 182 attached to the arm garment 160. Therefore, the hand strap 180 may define an opening between the interior surface 171 of the arm garment 160 and the hand strap 180. In other words, the middle section of the hand strap 180 (e.g., between the first and second ends 181, 182) may be spaced apart from the arm garment 160 such that the user may hold onto the hand strap 180.

The hand strap 180 may be configured to be gripped or held by the user such that the distal end region 162 of the arm garment 160 (e.g., proximate the hand of the user) maintains position relative to the arm and a remainder of the arm garment 160 (e.g., proximate the proximal end region 163) may be positionable (e.g., rotatable) relative to the arm. In other words, holding the hand strap 180 may properly position the hand relative to the arm garment 160 and the arm garment 160 may then be adjusted (e.g., rotationally about the arm) into a correct position relative to the arm. Specifically, the proximal end region 163 of the arm garment 160 may be twisted to the appropriate position while the distal end region 162 of the arm garment 160 remains in place.

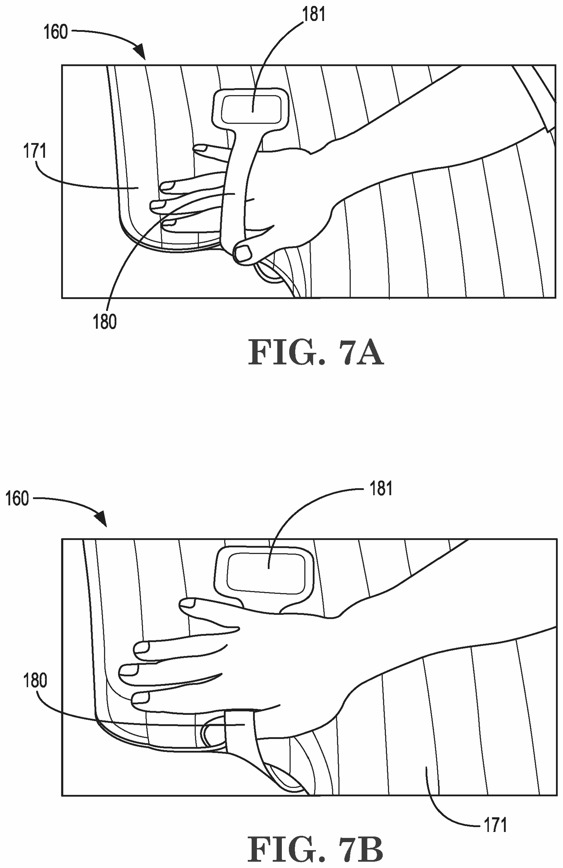

The hand strap 180 of the arm garment 160 may be positioned relative to the arm garment 160 in such a way to allow the user to naturally hold on to the hand strap 180. For example, the hand strap 180 may extend transverse to (e.g., at an angle to) the longitudinal axis 161 to align with the palm of the hand. Therefore, the hand strap 180 may be held by the user in a couple of different ways. For example, as shown in FIG. 7A, the four fingers (excluding the thumb) may be positioned between the hand strap 180 and the arm garment 160 while the thumb remains outside of the hand strap 180. Also, for example, as shown in FIG. 7B, the four fingers (excluding the thumb) may be positioned over the hand strap 180 with the thumb positioned between the hand strap 180 and the arm garment 160.

Further, the hand strap 180 may be located proximate the distal end region 162 of the arm garment 160 to land over the hand of the user. For example, in some embodiments, the hand strap 180 may be at least 2 inches from the distal end 164 of the arm garment 160. Also, the hand strap 180 may be coupled to the arm garment 160 in any suitable way. For example, as shown in FIGS. 6A and 6B, the first end 181 of the hand strap 180 may be attached to an interior surface 171 (e.g., as shown FIG. 6A) and the second end 182 of the hand strap 180 may be attached to the exterior surface 172 (e.g., as shown in FIG. 6B). Specifically, in one or more embodiments, the hand strap 180 may be located adjacent the cutout section 166 such that the hand strap 180 extends from the interior surface 171 to the exterior surface 172, and is attached to each, at the cutout section 166. By connecting the hand strap 180 to the exterior surface 172 of the arm garment 160, the material or fabric of the arm garment 160 may be twisted and smoothed over the top of the hand in the arm garment 160 (e.g., such that that the arm garment 160 may be wrapped around the hand and overlap without interference.

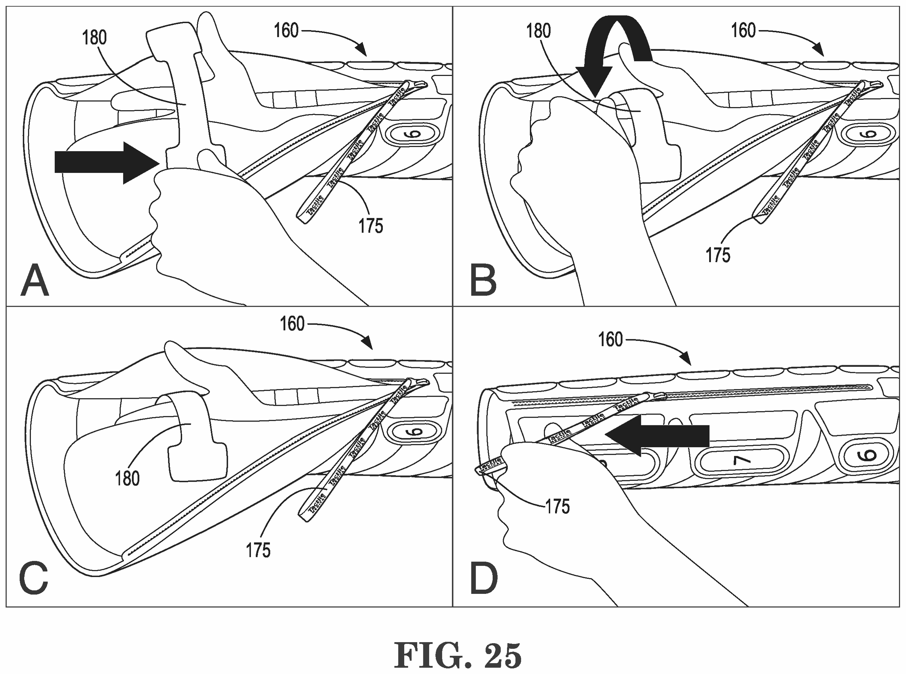

In one or more embodiments, the arm garment 160 may also include a zipper 175 extending from a distal end 164 of the arm garment 160 along the longitudinal axis 161 (e.g., as shown in FIG. 6B). The zipper 175 may be configured to couple a first region of the arm garment 160 and a second region of the arm garment 160 proximate the distal end region 162. As described previously, the zipper 175 may be used when a user dons a wrapped arm garment 160 (e.g., which forms a loose cylinder) by inserting an arm through an end opening of the arm garment 160. For example, by unzipping the zipper 175 located at the distal end region 162 of the arm garment 160, the user may more easily insert the arm into the arm garment 160 because of the expanded distal end region 162. After the arm garment 160 is in a proper position relative to the arm (e.g., using the hand strap 180), the zipper 175 may be zipped to decrease the size of the distal end region 162 of the arm garment 160 such that the arm garment 160 is in contact with the arm inserted therein (e.g., so that pressure may be applied to the arm without the garment pushing away from the arm). For example, FIG. 25 illustrates the hand strap 180 extending over the hand of the user and zipping the zipper 175 to secure the arm garment 160 into position. In one or more embodiments, the zipper 175 may include zipper straps to help the user hold onto and move the zipper 175.

In one or more embodiments, the arm garment 160 may include a stiffener rod 185 extending adjacent to the zipper 175 along the longitudinal axis 161 (e.g., on both sides of the zipper 175). The stiffener rods 185 are shown in dashed lines along each side of the zipper 175 in FIG. 6B. A stiffener rod 185 is also shown separate from the arm garment 160 in FIG. 6B. The stiffener rod 185 may help to add rigidity to the portions of the arm garment 160 next to the zipper 175 such that it is easier for the user to zip and unzip without causing the arm garment 160 to buckle. In other words, the stiffener rod 185 may make it such that the arm garment 160 may be zipped and unzipped using one hand. The stiffener rod 185 may be positioned between an interior surface 171 of the arm garment 160 and an exterior surface 172 of the arm garment 160 (e.g., embedded in the arm garment 160 material). Further, the stiffener rod 185 may extend from the distal end 164 for a distance that is less than the length of the zipper 175 extending from the distal end 164. Specifically, the stiffener rod 185 may define a length of about 7 inches.

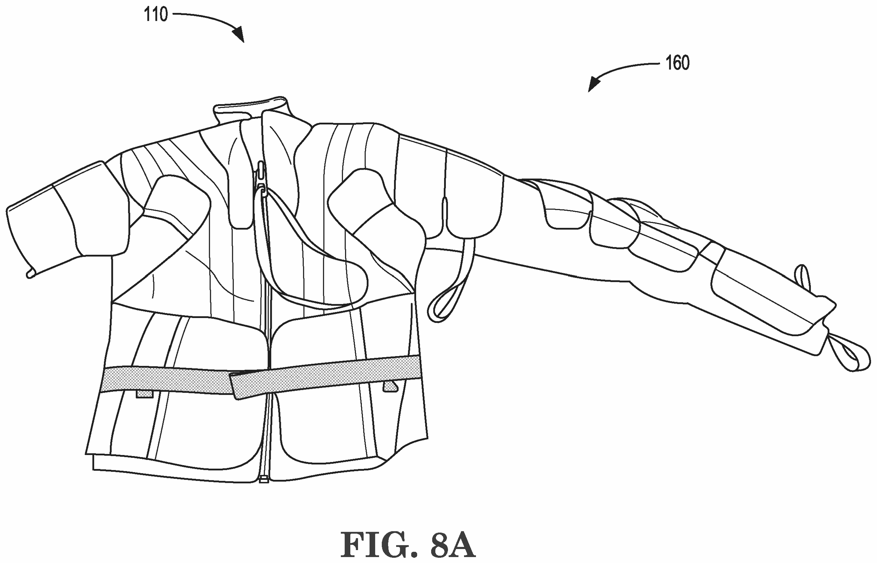

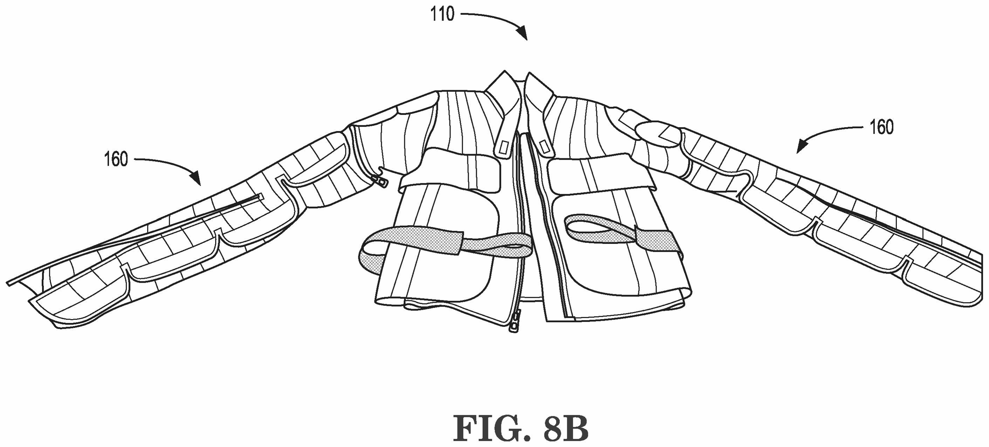

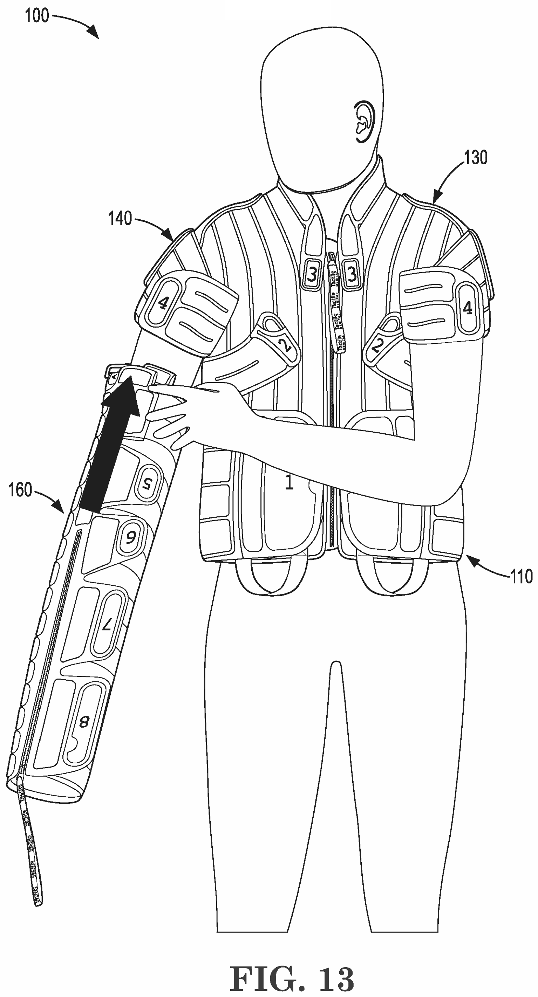

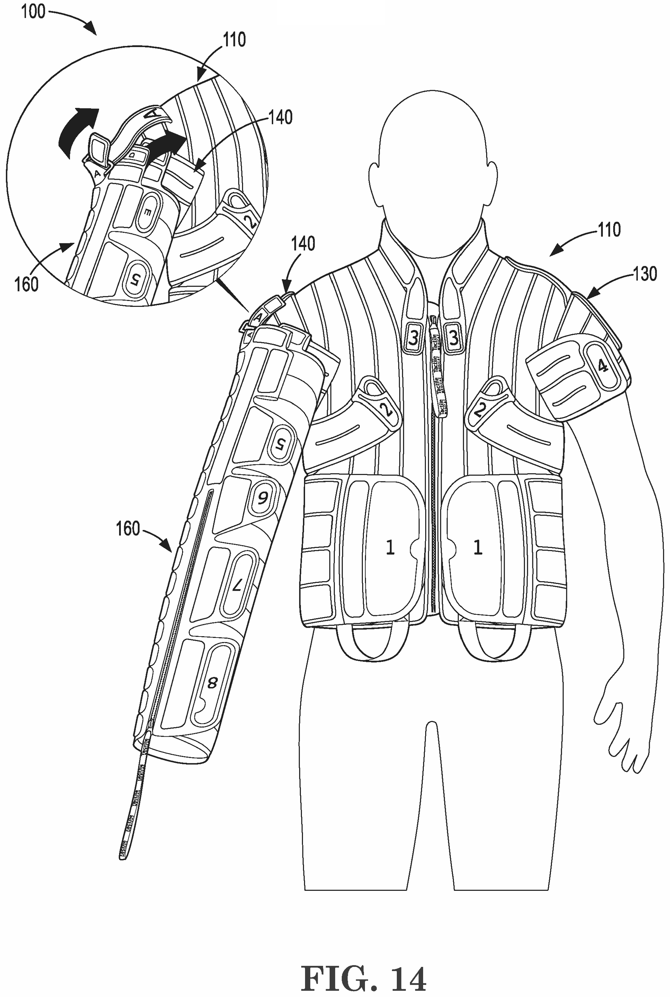

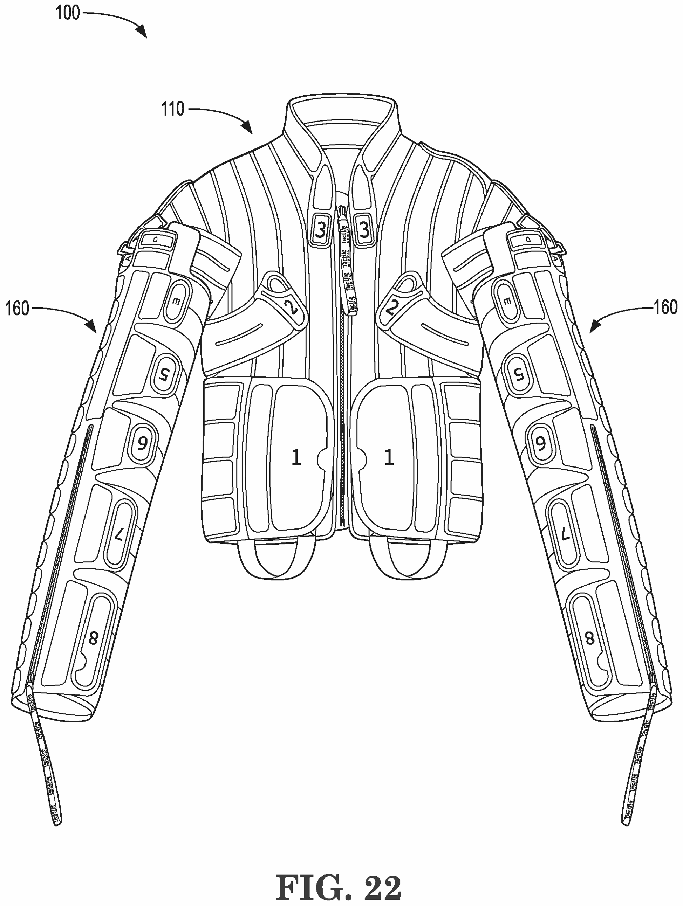

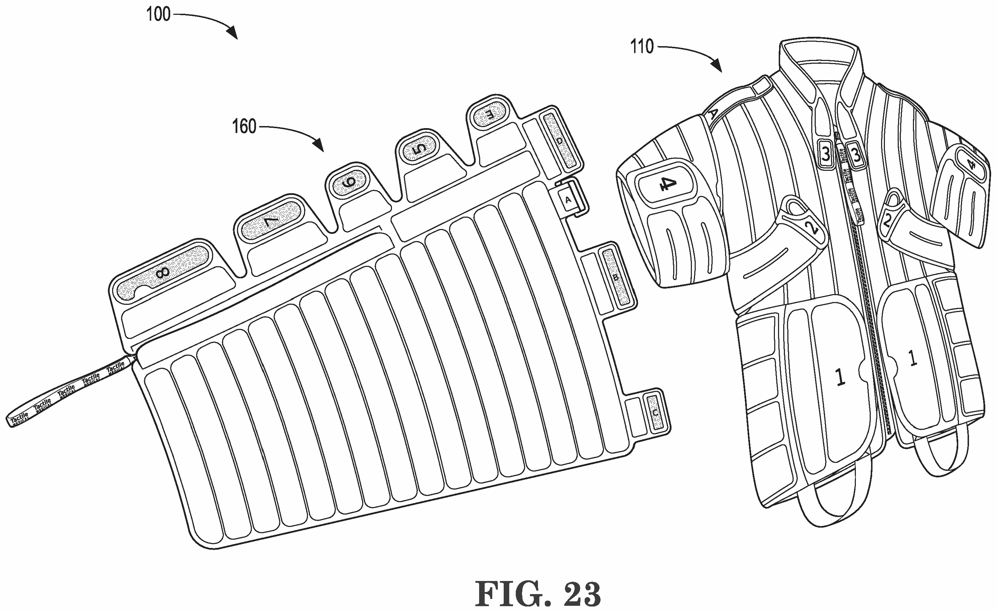

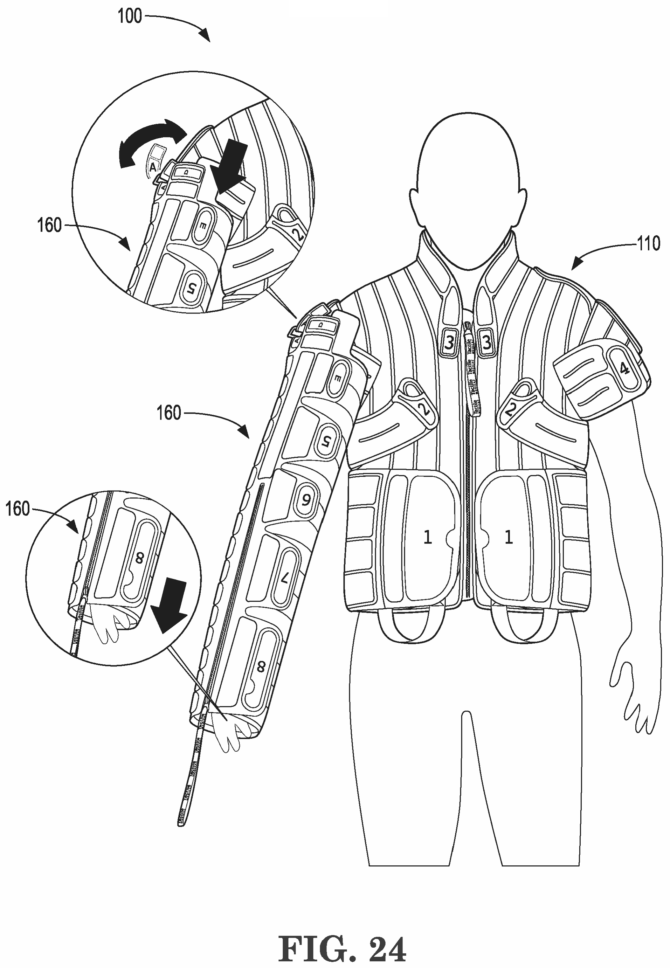

Furthermore, the arm garment 160 may be configured to be used in combination with the upper body garment 110. FIG. 23 illustrates the upper body garment 110 in proximity to the arm garment 160 in an open/unwrapped configuration. For example, the upper body garment 110 may be used with a single arm garment 160 (e.g., as shown in FIG. 8A, 15 and 23) or two arm garments 160 (e.g., as shown in FIGS. 8B and 22). FIG. 23 illustrates the single arm garment 160 in an unwrapped configuration. FIG. 13 illustrates the user pulling the arm garment 160 up the arm (e.g., donning the arm garment 160) towards the upper body garment 110. At least a portion of the arm garment 160 may overlap with at least a portion of the upper body garment 110 when the arm garment 160 is in position. For example, the arm garment 160 may overlap the upper arm garment portion 130, 140 of the upper body garment 110. Further, in one or more embodiments, the arm garment 160 may be coupled to the upper body garment 110 (e.g., as shown in FIGS. 14 and 24). For example, the arm garment 160 may be coupled to the upper body garment 110 using any suitable types of connection such as, e.g., hook-and-loop fasteners, zippers, a connection tab, buttons, clasps, magnets, etc.

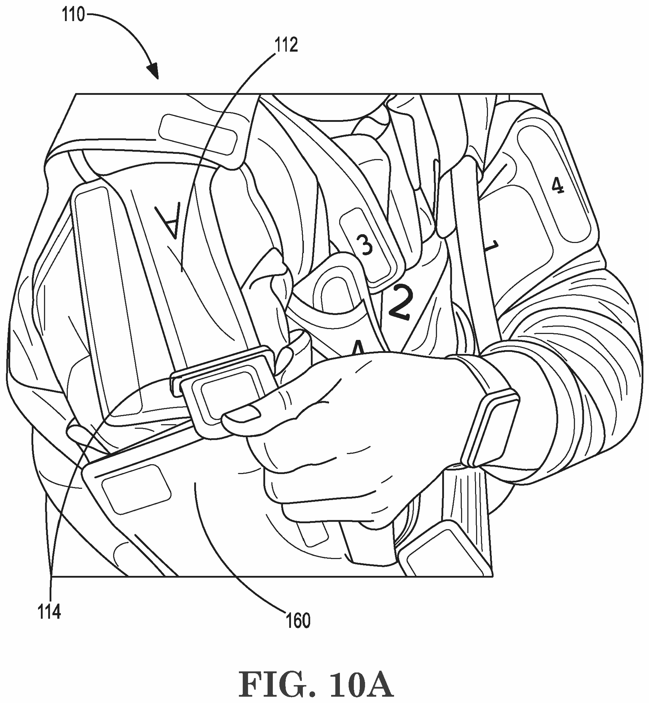

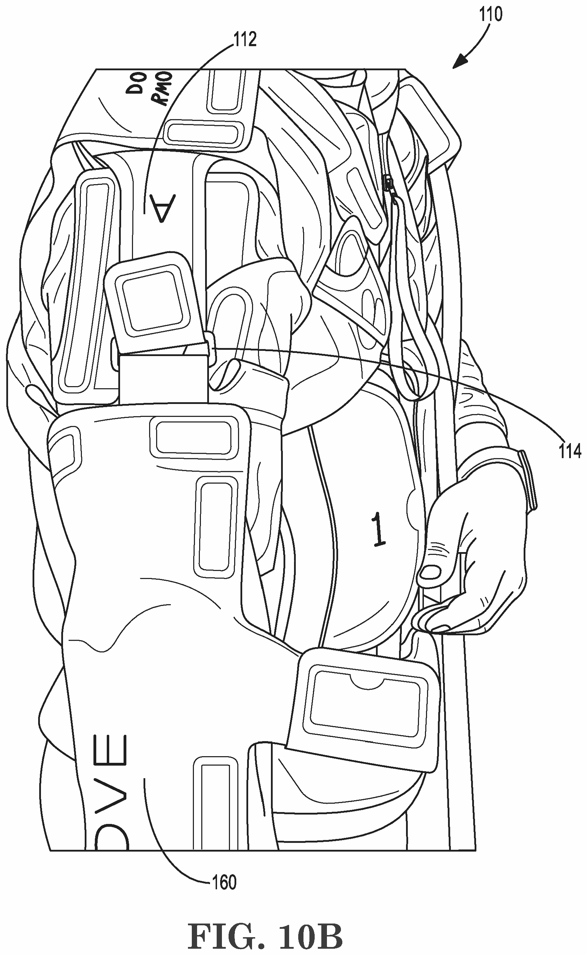

For example, in one or more embodiments, the upper body garment 110 may include a connection strap 112 configured to attach the arm garment 160 to the upper body garment 110 (e.g., as shown in FIGS. 10A and 10B). Specifically, the connection strap 112 may lay on the top over the shoulder of the upper body garment 110. Further, the connection strap 112 may be threaded through a ring 114 (e.g., plastic ring) attached to the arm garment 160. The ring 114 may be located at the proximal end region 163 (e.g., at the proximal end) of the arm garment 160. The connection strap 112 may extend through the ring 114 and attached to itself (e.g., by folding over) or some other portion of the compression garment to affix and align (e.g., longitudinally and rotationally) the arm garment 160 to the upper body garment 110 (e.g., to align the arm and bicep portions of each garment). Additionally, in one or more embodiments, the connection strap 112 may be tightened to move the arm garment 160 higher up on the sleeve of the upper body garment 110.

In one or more embodiments, the compression garment system may include color coded attachment tabs to assist the user in properly positioning and attaching portions of the compression garment to the body. For example, the attachment tabs 109 of the upper body garment 110 may include a variety of different colors and portions on the upper body garment 110 (at which the attachment tabs 109 are to be attached) may include the same corresponding colors to assist in aligning the attachment tabs 109. Similarly, the attachment tabs 169 of the arm garment 160 may include a variety of different colors and portions of the arm garment 160 (at which the attachment tabs 169 are to be attached) may include the same corresponding colors to assist in aligning the attachment tabs 169. In one or more embodiments, the compression garment system may similarly use numbers instead color coding.

A cross-section of a portion 800 of an exemplary garment including one or more chambers 801 which may be used in providing any of the garments described herein is shown in FIG. 9. The garment portion 800 may define an exterior surface 802 configured to face the exterior, e.g., away from a user when wearing the garment portion 800, and an opposing interior surface 803 configured to face the interior, e.g., towards a user wearing the garment portion 800. The interior surface 803 may be configured to be positioned closer to the human body than the exterior surface 802 when the garment portion 800 is positioned on the body. As shown, the garment portion 800 defines a plurality of chambers configured and corresponding to pressure applying regions. Each of the chambers 801 defines a volume 805 that may be separated in any way that isolates the volume 805 of a chamber from the volumes of the other chambers 801 (e.g., such that the chambers may be inflated/deflated separately from one another). For example, the volumes 805 of the chambers 801 may be separated by welds 815, e.g., welds between one or more layers of the garment portion 800 as will be further described herein. The volumes, or cavities, 805 defined by, or in each, of the chambers 801 may be configured to receive a fluid. The fluid may be received from a source (e.g., from pump 103 shown in FIGS. 1A and 5) to apply pressure at a pressure applying region of the garment to a body portion when garment portion 800 is worn by a user. For example, fluid may be directed to each of the volumes 805 of the chambers 801 in a sequential or non-sequential manner.

Further, each of the various pressure applying regions described herein may include, e.g., one of the one or more chambers 801 or a plurality of the chambers 801. In one or more embodiments, different pressure applying regions described herein may include, e.g., the same one or more chambers, but may, e.g., be positioned at different locations on the garment.

The garment portion 800 may include one or more layers from the exterior surface 802 to the interior surface 803. For example, the exterior facing layer 806, or the layer defining the exterior surface 802, may include one or more fabric materials so as to define a "hook" surface on the exterior surface 802 for coupling to a "loop" surface or material forming, or defining, a "hook-and-loop" fastener. The exterior surface of the exemplary garment portions described herein may be partially or completely defined by a "hook" surface for use in a "hook-and-loop" fastener.

A foam layer 807 may be adjacent to the exterior facing layer 806, and then a polymer layer 808 (e.g., polyurethane, polyvinyl, etc.) may be located adjacent the foam layer 807 facing the volume 805 of the chamber 801. The interior side of the garment portion 800 may be similar to the exterior side except that, instead of an exterior facing layer, the foam layer 807 may be adjacent a fabric layer 809 configured to be located adjacent the torso of a body. Furthermore, in one or more embodiments, the layers that form the garment portion 800 may be inelastic. In other words, the garment portion may not be stretched and return to its original size or shape thereafter.

A plan view of the components of the upper body garment is illustrated in FIGS. 12A and 12B. Specifically, the exterior of the upper body garment is shown in FIG. 12A and the interior of the upper body garment is shown in FIG. 12B.

Unless otherwise indicated, all numbers expressing feature sizes, amounts, and physical properties used in the specification and claims are to be understood as being modified in all instances by the term "about." Accordingly, unless indicated to the contrary, the numerical parameters set forth in the foregoing specification and attached claims are approximations that can vary depending upon the desired properties sought to be obtained by those skilled in the art utilizing the teachings disclosed herein. The use of numerical ranges by endpoints includes all numbers within that range (e.g. 1 to 5 includes 1, 1.5, 2, 2.75, 3, 3.80, 4, and 5) and any range within that range.

Particular materials and dimensions thereof recited in the disclosed examples, as well as other conditions and details, should not be construed to unduly limit this disclosure.

## Claims

1. A compression garment system comprising:
an upper body garment (110) positionable proximate an upper body of a user, wherein the upper body garment (110) comprises a torso garment portion (120), a left upper arm garment portion (130), and a right upper arm garment portion (140), wherein the upper body garment (110) comprises a plurality of pressure applying regions controllable to apply pressure to a plurality of portions of the upper body, wherein the plurality of pressure applying regions comprises a plurality of torso pressure applying regions (122), at least one left upper arm pressure applying region (132), and at least one right upper arm pressure applying region (142); and
a controller (102) operably coupled to the upper body garment (110) to control pressure applied by the plurality of pressure applying regions to the upper body, wherein the plurality of torso pressure applying regions (122) are configured to apply pressure to a plurality of portions of a torso, wherein the at least one left upper arm pressure applying region (132) is configured to apply pressure to a portion of a left upper arm, and wherein the at least one right upper arm pressure applying region (142) is configured to apply pressure to a portion of a right upper arm.

2. The compression garment system of claim 1, wherein the at least one left upper arm pressure applying region (132) comprises two left upper arm pressure applying regions and the at least one right upper arm pressure applying region (142) comprises two right upper arm pressure applying regions.

3. The compression garment system of any one of claims 1-2, further comprising an arm garment (160) positionable proximate an arm of the user, wherein the arm garment (160) comprises a plurality of arm pressure applying regions (165) controllable to apply pressure to a plurality of portions of the arm.

4. The compression garment system of any one of claims 1-3, wherein the torso garment portion (120) comprises:
a left torso portion (123) to extend from a posterior of the torso across a left side of the torso to an anterior of the torso,
a right torso portion (124) to extend from the posterior of the torso across a right side of the torso to the anterior of the torso, wherein the right torso portion (124) is removably couplable to the left torso portion (123) proximate the anterior of the torso,
a posterior torso portion (121) positionable proximate the posterior of the torso and coupled to the left and the right torso portions (123, 124) proximate a neck region of the torso,
a left wraparound portion (126) extending from the posterior torso portion (121) to extend around the left side of the torso to the anterior of the torso, wherein the left wraparound portion (126) is removably couplable to at least the left torso portion (123) to tighten the torso garment portion (120) about the torso, and
a right wraparound portion (127) extending from the posterior torso portion (121) to extend around the right side of the torso to the anterior of the torso, wherein the right wraparound portion (127) is removably couplable to at least the right torso portion (124) to tighten the torso garment portion (120) about the torso.

5. The compression garment system of claim 4, wherein the torso garment portion (120) further comprises a left strap (134) and a right strap (135), wherein the left strap (134) is attached to a portion of the left torso portion (123), extends inwards towards a central portion of the posterior torso portion (121), and then outwards around the left torso portion (123), wherein the right strap (135) is attached to a portion of the right torso portion (124), extends inwards towards the central portion of the posterior torso portion (121), and then outwards around the right torso portion (124), wherein the left and right straps (134, 135) are movably connected to one another proximate the central portion of the posterior torso portion (121).

6. The compression garment system of claim 5, wherein the left strap (134) is removably couplable to the left torso portion (123) proximate the anterior of the torso and the right strap (135) is removably couplable to the right torso portion (124) proximate the anterior of the torso.

7. The compression garment system of claim 5, wherein the torso garment portion (120) further comprises a cover (129) attached to an exterior surface of the posterior torso portion (121) over the left and right straps (134, 135) proximate the central portion of the posterior torso portion (121).

8. The compression garment system of claim 5, wherein each of the left and right straps (134, 135) extend from a proximal end (136) attached to the left and right torso portions (123, 124), respectively, and a distal end (137) configured to be removably couplable to the left and right torso portions (123, 124), respectively, wherein the distal ends of the left and right straps (134, 135) comprise a loop configured to be manipulated by a hand of the user.

9. The compression garment system of any one of claims 1-8, wherein the upper body garment (110) further comprises at least one underarm strap (150) positionable proximate an underarm region of the user between the torso garment portion (120) and the at least one of the upper arm garment portions (130, 140), wherein the plurality of pressure applying regions comprises at least one underarm pressure applying region (155) configured to apply pressure to a portion of the underarm region.

10. The compression garment system of claim 9, wherein the at least one underarm strap (150) comprises a left underarm strap positionable between the left upper arm garment portion (130) and the torso garment portion (120), wherein the at least one underarm strap (150) comprises a right underarm strap positionable between the right upper arm garment portion (140) and the torso garment portion (120).

11. The compression garment system of claim 9, wherein the at least one underarm strap (150) is configured to extend between a posterior of the upper body garment (110) and an anterior of the upper body garment (110), through the underarm region of the user.

12. The compression garment system of claim 3, wherein the arm garment (160) extends between a distal end region (162) and a proximal end region (163) along a longitudinal axis (161), wherein the arm garment (160) comprises a hand strap (180) attached to arm garment proximate the distal end region (162), wherein the hand strap (180) defines an opening between an interior surface of the arm garment (160) and the hand strap (180), wherein the hand strap (180) is configured to be gripped by the user such that the distal end region (162) of the arm garment (160) maintains position relative to the arm and a remainder of the arm garment (160) is positionable relative to the arm, and wherein the controller (102) is operably coupled to the arm garment (160) to control pressure applied by the plurality of arm pressure applying regions (165) to the arm.

13. The compression garment system of claim 12, wherein the hand strap (180) extends between a first end (181) and a second end (182), wherein the first end (181) of the hand strap (180) is attached to an interior surface of the arm garment (160) and the second end (182) of the hand strap (180) is attached to an exterior surface of the arm garment (160).

14. The compression garment system of claim 12, wherein the hand strap (180) extends transverse to the longitudinal axis (161).

15. The compression garment system of claim 12, wherein the arm garment (160) further comprises:
a zipper (175) extending from a distal end of the arm garment (160) along the longitudinal axis (161), wherein the zipper (175) is configured to couple a first region of the arm garment (160) and a second region of the arm garment (160) proximate the distal end region (162);
a stiffener rod (185) extending adjacent to the zipper (175) along the longitudinal axis (161); and
a tubing harness operably coupled to the arm garment (160) at an elbow region of the arm garment (160).

## Patentansprüche

1. Kompressionsbekleidungssystem, umfassend:
eine Oberkörperbekleidung (110), welche proximal zu einem Oberkörper eines Benutzers positionierbar ist, wobei die Oberkörperbekleidung (110) einen Rumpfbekleidungsabschnitt (120), einen linken Oberarmbekleidungsabschnitt (130) und einen rechten Oberarmbekleidungsabschnitt (140) umfasst, wobei die Oberkörperbekleidung (110) eine Mehrzahl von Druckaufbringungsbereichen umfasst, welche steuerbar ist, um Druck auf eine Mehrzahl von Abschnitten des Oberkörpers aufzubringen, wobei die Mehrzahl von Druckaufbringungsbereichen eine Mehrzahl von Rumpfdruckaufbringungsbereichen (122), wenigstens einen linken Oberarmdruckaufbringungsbereich (132) und wenigstens einen rechten Oberarmdruckaufbringungsbereich (142) umfasst; und
eine Steuereinheit (102), welche betriebsfähig mit der Oberkörperbekleidung (110) gekoppelt ist, um einen Druck zu steuern, welcher durch die Mehrzahl von Druckaufbringungsbereichen auf den Oberkörper aufgebracht wird, wobei die Mehrzahl von Rumpfdruckaufbringungsbereichen (122) dazu eingerichtet ist, Druck auf eine Mehrzahl von Abschnitten eines Rumpfes aufzubringen, wobei der wenigstens eine linke Oberarmdruckaufbringungsbereich (132) dazu eingerichtet ist, Druck auf einen Abschnitt eines linken Oberarms aufzubringen, und wobei der wenigstens eine rechte Oberarmdruckaufbringungsbereich (142) dazu eingerichtet ist, Druck auf einen Abschnitt eines rechten Oberarms aufzubringen.

2. Kompressionsbekleidungssystem nach Anspruch 1, wobei der wenigstens eine linke Oberarmdruckaufbringungsbereich (132) zwei linke Oberarmdruckaufbringungsbereiche umfasst und der wenigstens eine rechte Oberarmdruckaufbringungsbereich (142) zwei rechte Oberarmdruckaufbringungsbereiche umfasst.

3. Kompressionsbekleidungssystem nach einem der Ansprüche 1 bis 2, ferner umfassend eine Armbekleidung (160), welche proximal zu einem Arm des Benutzers positionierbar ist, wobei die Armbekleidung (160) eine Mehrzahl von Armdruckaufbringungsbereichen (165) umfasst, welche steuerbar ist, um Druck auf eine Mehrzahl von Abschnitten des Arms aufzubringen.

4. Kompressionsbekleidungssystem nach einem der Ansprüche 1 bis 3, wobei der Rumpfbekleidungsabschnitt (120) umfasst:
einen linken Rumpfabschnitt (123), welcher dazu eingerichtet ist, sich von einer Rückseite des Rumpfes über eine linke Seite des Rumpfes zu einer Vorderseite des Rumpfes zu erstrecken,
einen rechten Rumpfabschnitt (124), welcher dazu eingerichtet ist, sich von der Rückseite des Rumpfes über eine rechte Seite des Rumpfes zu der Vorderseite des Rumpfes zu erstrecken, wobei der rechte Rumpfabschnitt (124) proximal zu der Vorderseite des Rumpfes lösbar mit dem linken Rumpfabschnitt (123) koppelbar ist,
einen rückseitigen Rumpfabschnitt (121), welcher proximal zu der Rückseite des Rumpfes positionierbar und proximal zu einem Halsbereich des Rumpfes mit dem linken und dem rechten Rumpfabschnitt (123, 124) gekoppelt ist,
einen linken Umwicklungsabschnitt (126), welcher sich von dem rückseitigen Rumpfabschnitt (121) erstreckt, um sich um die linke Seite des Rumpfes zu der Vorderseite des Rumpfes zu erstrecken, wobei der linke Umwicklungsabschnitt (126) wenigstens mit dem linken Rumpfabschnitt (123) lösbar koppelbar ist, um den Rumpfbekleidungsabschnitt (120) um den Rumpf zu straffen, und
einen rechten Umwicklungsabschnitt (127), welcher sich von dem rückseitigen Rumpfabschnitt (121) erstreckt, um sich um die rechte Seite des Rumpfes zu der Vorderseite des Rumpfes zu erstrecken, wobei der rechte Umwicklungsabschnitt (127) wenigstens mit dem rechten Rumpfabschnitt (124) lösbar koppelbar ist, um den Rumpfbekleidungsabschnitt (120) um den Rumpf zu straffen.

5. Kompressionsbekleidungssystem nach Anspruch 4, wobei der Rumpfbekleidungsabschnitt (120) ferner einen linken Gurt (134) und einen rechten Gurt (135) umfasst, wobei der linke Gurt (134) an einem Abschnitt des linken Rumpfabschnitts (123) befestigt ist, sich einwärts zu einem zentralen Abschnitt des rückseitigen Rumpfabschnitts (121) und dann auswärts um den linken Rumpfabschnitt (123) herum erstreckt, wobei der rechte Gurt (135) an einem Abschnitt des rechten Rumpfabschnitts (124) befestigt ist, sich einwärts zu dem zentralen Abschnitt des rückseitigen Rumpfabschnitts (121) und dann auswärts um den rechten Rumpfabschnitt (124) herum erstreckt, wobei der linke und der rechte Gurt (134, 135) proximal zu dem zentralen Abschnitt des rückseitigen Rumpfabschnitts (121) beweglich miteinander verbunden sind.

6. Kompressionsbekleidungssystem nach Anspruch 5, wobei der linke Gurt (134) proximal zu der Vorderseite des Rumpfes lösbar mit dem linken Rumpfabschnitt (123) koppelbar ist und der rechte Gurt (135) proximal zu der Vorderseite des Rumpfes lösbar mit dem rechten Rumpfabschnitt (124) koppelbar ist.

7. Kompressionsbekleidungssystem nach Anspruch 5, wobei der Rumpfbekleidungsabschnitt (120) ferner eine Abdeckung (129) umfasst, welche an einer Außenfläche des rückseitigen Rumpfabschnitts (121) über dem linken und dem rechten Gurt (134, 135) proximal zu dem zentralen Abschnitt des rückseitigen Rumpfabschnitts (121) befestigt ist.

8. Kompressionsbekleidungssystem nach Anspruch 5, wobei sich jeder der linken und rechten Gurte (134, 135) von einem proximalen Ende (136), welches an dem linken bzw. rechten Rumpfabschnitt (123, 124) befestigt ist, zu einem distalen Ende (137) erstreckt, welches dazu eingerichtet ist, lösbar mit dem linken bzw. rechten Rumpfabschnitt (123, 124) koppelbar zu sein, wobei die distalen Enden des linken und des rechten Gurtes (134, 135) eine Schlaufe umfassen, welche dazu eingerichtet ist, von einer Hand des Benutzers betätigt zu werden.

9. Kompressionsbekleidungssystem nach einem der Ansprüche 1 bis 8, wobei die Oberkörperbekleidung (110) ferner wenigstens einen Achselgurt (150) umfasst, welcher proximal zu einem Achselbereich des Benutzers zwischen dem Rumpfbekleidungsabschnitt (120) und wenigstens einem der Oberarmbekleidungsabschnitte (130, 140) positionierbar ist, wobei die Mehrzahl von Druckaufbringungsbereichen wenigstens einen Achseldruckaufbringungsbereich (155) umfasst, welcher dazu eingerichtet ist, Druck auf einen Abschnitt des Achselbereichs aufzubringen.

10. Kompressionsbekleidungssystem nach Anspruch 9, wobei der wenigstens eine Achselgurt (150) einen linken Achselgurt umfasst, welcher zwischen dem linken Oberarmbekleidungsabschnitt (130) und dem Rumpfbekleidungsabschnitt (120) positionierbar ist, wobei der wenigstens eine Achselgurt (150) einen rechten Achselgurt umfasst, welcher zwischen dem rechten Oberarmbekleidungsabschnitt (140) und dem Rumpfbekleidungsabschnitt (120) positionierbar ist.

11. Kompressionsbekleidungssystem nach Anspruch 9, wobei der wenigstens eine Achselgurt (150) dazu eingerichtet ist, sich durch den Achselbereich des Benutzers zwischen einer Rückseite der Oberkörperbekleidung (110) und einer Vorderseite der Oberkörperbekleidung (110) zu erstrecken.

12. Kompressionsbekleidungssystem nach Anspruch 3, wobei die Armbekleidung (160) sich entlang einer Longitudinalachse (161) zwischen einem distalen Endbereich (162) und einem proximalen Endbereich (163) erstreckt, wobei die Armbekleidung (160) einen Handgurt (180) umfasst, welcher proximal zu dem distalen Endbereich (162) an der Armbekleidung (160) befestigt ist, wobei der Handgurt (180) eine Öffnung zwischen einer Innenfläche der Armbekleidung (160) und dem Handgurt (180) definiert, wobei der Handgurt (180) dazu eingerichtet ist, von dem Benutzer gegriffen zu werden, sodass der distale Endbereich (162) der Armbekleidung (160) seine Position relativ zu dem Arm beibehält und ein verbleibender Teil der Armbekleidung (160) relativ zu dem Arm positionierbar ist, und wobei die Steuereinheit (102) betriebsfähig mit der Armbekleidung (160) gekoppelt ist, um den Druck zu steuern, welcher durch die Mehrzahl von Armdruckaufbringungsbereichen (165) auf den Arm aufgebracht wird.

13. Kompressionsbekleidungssystem nach Anspruch 12, wobei der Handgurt (180) sich zwischen einem ersten Ende (181) und einem zweiten Ende (182) erstreckt, wobei das erste Ende (181) des Handgurts (180) an einer Innenfläche der Armbekleidung (160) befestigt ist und das zweite Ende (182) des Handgurts (180) an einer Außenfläche der Armbekleidung (160) befestigt ist.

14. Kompressionsbekleidungssystem nach Anspruch 12, wobei sich der Handgurt (180) quer zu der Longitudinalachse (161) erstreckt.

15. Kompressionsbekleidungssystem nach Anspruch 12, wobei die Armbekleidung (160) ferner umfasst:
einen Reißverschluss (175), welcher sich von einem distalen Ende der Armbekleidung (160) entlang der Longitudinalachse (161) erstreckt, wobei der Reißverschluss (175) dazu eingerichtet ist, einen ersten Bereich der Armbekleidung (160) und einen zweiten Bereich der Armbekleidung (160) proximal zu dem distalen Endbereich (162) zu koppeln;
einen Versteifungsstab (185), welcher sich entlang der Longitudinalachse (161) angrenzend an den Reißverschluss (175) erstreckt; und
ein Schlauchbündel, welches betriebsfähig mit der Armbekleidung (160) an einem Ellenbogenbereich der Armbekleidung (160) gekoppelt ist.

## Revendications

1. Système de vêtement de compression comprenant :
un vêtement pour le haut du corps (110) pouvant être positionné près du haut du corps d'un utilisateur, dans lequel le vêtement pour le haut du corps (110) comprend une portion de vêtement pour le torse (120), une portion de vêtement pour le haut du bras gauche (130), et une portion de vêtement pour le haut du bras droit (140), dans lequel le vêtement pour le haut du corps (110) comprend une pluralité de régions d'application de pression pouvant être contrôlées pour appliquer une pression à une pluralité de portions du haut du corps, dans lequel la pluralité de régions d'application de pression comprend une pluralité de régions d'application de pression au torse (122), au moins une région d'application de pression au haut du bras gauche (132), et au moins une région d'application de pression au haut du bras droit (142) ; et
un dispositif de commande (102) fonctionnellement accouplé au vêtement pour le haut du corps (110) pour commander la pression appliquée par la pluralité de régions d'application de pression au haut du corps, dans lequel la pluralité de régions d'application de pression au torse (122) est configurées pour appliquer une pression à une pluralité de portions d'un torse, dans lequel l'au moins une région d'application de pression au haut du bras gauche (132) est configurée pour appliquer une pression à une portion du haut du bras gauche, et dans lequel l'au moins une région d'application de pression au haut du bras droit (142) est configurée pour appliquer une pression à une portion du haut du bras droit.

2. Système de vêtement de compression selon la revendication 1, dans lequel l'au moins une région d'application de pression au haut du bras gauche (132) comprend deux régions d'application de pression au haut du bras gauche et l'au moins une région d'application de pression au haut du bras droit (142) comprend deux régions d'application de pression au haut du bras droit.

3. Système de vêtement de compression selon l'une quelconque des revendications 1 à 2, comprenant en outre un vêtement pour le bras (160) pouvant être positionné près d'un bras de l'utilisateur, dans lequel le vêtement pour le bras (160) comprend une pluralité de régions d'application de pression au bras (165) pouvant être contrôlées pour appliquer une pression à une pluralité de portions du bras.

4. Système de vêtement de compression selon l'une quelconque des revendications 1 à 3, dans lequel la portion de vêtement pour le torse (120) comprend :
une portion gauche du torse (123) pour s'étendre depuis l'arrière du torse à travers un côté gauche du torse vers l'avant du torse,
une portion droite du torse (124) pour s'étendre depuis l'arrière du torse à travers un côté droit du torse vers l'avant du torse, dans lequel la portion droite du torse (124) peut être accouplée de manière amovible à la portion gauche du torse (123) près de l'avant du torse,
une portion arrière du torse (121) pouvant être positionnée près de l'arrière du torse et accouplée aux portions gauche et droite du torse (123, 124) près d'une région de cou du torse,
une portion enveloppante gauche (126) s'étendant depuis la portion arrière du torse (121) pour s'étendre autour du côté gauche du torse vers l'avant du torse, dans lequel la portion enveloppante gauche (126) peut être accouplée de manière amovible à au moins la portion gauche du torse (123) pour resserrer la portion de vêtement pour le torse (120) autour du torse, et
une portion enveloppante droite (127) s'étendant depuis la portion arrière du torse (121) pour s'étendre autour du côté droit du torse vers l'avant du torse, dans lequel la portion enveloppante droite (127) peut être accouplée de manière amovible à au moins la portion droite du torse (124) pour resserrer la portion de vêtement pour le torse (120) autour du torse.

5. Système de vêtement de compression selon la revendication 4, dans lequel la portion de vêtement pour le torse (120) comprend en outre une sangle gauche (134) et une sangle droite (135), dans lequel la sangle gauche (134) est fixée à une portion de la portion gauche du torse (123), s'étend vers l'intérieur vers une portion centrale de la portion arrière du torse (121), et ensuite vers l'extérieur autour de la portion gauche du torse (123), dans lequel la sangle droite (135) est fixée à une portion de la portion droite du torse (124), s'étend vers l'intérieur vers la portion centrale de la portion arrière du torse (121), et ensuite vers l'extérieur autour de la portion droite du torse (124), dans lequel les sangles gauche et droite (134, 135) sont reliées de manière mobile l'une à l'autre près de la portion centrale de la portion arrière du torse (121).

6. Système de vêtement de compression selon la revendication 5, dans lequel la sangle gauche (134) peut être accouplée de manière amovible à la portion gauche du torse (123) près de l'avant du torse et la sangle droite (135) peut être accouplée de manière amovible à la portion droite du torse (124) près de l'avant du torse.

7. Système de vêtement de compression selon la revendication 5, dans lequel la portion de vêtement pour le torse (120) comprend en outre un cache (129) fixé à une surface extérieure de la portion arrière du torse (121) par-dessus les sangles gauche et droite (134, 135) près de la portion centrale de la portion arrière du torse (121).

8. Système de vêtement de compression selon la revendication 5, dans lequel chacune des sangles gauche et droite (134, 135) s'étend depuis une extrémité proximale (136) fixée aux portions gauche et droite du torse (123, 124), respectivement, et une extrémité distale (137) configurée pour pouvoir être accouplée de manière amovible aux portions gauche et droite du torse (123, 124), respectivement, dans lequel les extrémités distales des sangles gauche et droite (134, 135) comprennent une boucle configurée pour être manipulée par une main de l'utilisateur.

9. Système de vêtement de compression selon l'une quelconque des revendications 1 à 8, dans lequel le vêtement pour le haut du corps (110) comprend en outre au moins une sangle au niveau de l'aisselle (150) pouvant être positionnée près d'une région d'aisselle de l'utilisateur entre la portion de vêtement pour le torse (120) et l'au moins une des portions de vêtement pour le haut du bras (130, 140), dans lequel la pluralité de régions d'application de pression comprend au moins une région d'application de pression pour l'aisselle (155) configurée pour appliquer une pression à une portion de la région d'aisselle.

10. Système de vêtement de compression selon la revendication 9, dans lequel l'au moins une sangle au niveau de l'aisselle (150) comprend une sangle pour l'aisselle gauche pouvant être positionnée entre la portion de vêtement pour le haut du bras gauche (130) et la portion de vêtement pour le torse (120), dans lequel l'au moins une sangle au niveau de l'aisselle (150) comprend une sangle pour l'aisselle droite pouvant être positionnée entre la portion de vêtement pour le haut du bras droit (140) et la portion de vêtement pour le torse (120).

11. Système de vêtement de compression selon la revendication 9, dans lequel l'au moins une sangle au niveau de l'aisselle (150) est configurée pour s'étendre entre l'arrière du vêtement pour le haut du corps (110) et l'avant du vêtement pour le haut du corps (110), à travers la région d'aisselle de l'utilisateur.

12. Système de vêtement de compression selon la revendication 3, dans lequel le vêtement pour le bras (160) s'étend entre une région d'extrémité distale (162) et une région d'extrémité proximale (163) le long d'un axe longitudinal (161), dans lequel le vêtement pour le bras (160) comprend une sangle pour la main (180) fixée au vêtement pour le bras près de la région d'extrémité distale (162), dans lequel la sangle pour la main (180) définit une ouverture entre une surface intérieure du vêtement pour le bras (160) et la sangle pour la main (180), dans lequel la sangle pour la main (180) est configurée pour être saisie par l'utilisateur de sorte que la région d'extrémité distale (162) du vêtement pour le bras (160) maintient une position par rapport au bras et un reste du vêtement pour le bras (160) peut être positionné par rapport au bras, et dans lequel le dispositif de commande (102) est fonctionnellement accouplé au vêtement pour le bras (160) pour contrôler une pression appliquée par la pluralité de régions d'application de pression au bras (165) au bras.

13. Système de vêtement de compression selon la revendication 12, dans lequel la sangle pour la main (180) s'étend entre une première extrémité (181) et une seconde extrémité (182), dans lequel la première extrémité (181) de la sangle pour la main (180) est fixée à une surface intérieure du vêtement pour le bras (160) et la seconde extrémité (182) de la sangle pour la main (180) est fixée à une surface extérieure du vêtement pour le bras (160).

14. Système de vêtement de compression selon la revendication 12, dans lequel la sangle pour la main (180) s'étend transversalement à l'axe longitudinal (161).

15. Système de vêtement de compression selon la revendication 12, dans lequel le vêtement pour le bras (160) comprend en outre :
une fermeture à glissière (175) s'étendant depuis une extrémité distale du vêtement pour le bras (160) le long de l'axe longitudinal (161), dans lequel la fermeture à glissière (175) est configurée pour accoupler une première région du vêtement pour le bras (160) et une seconde région du vêtement pour le bras (160) près de la région d'extrémité distale (162) ;
une tige raidisseuse (185) s'étendant adjacente à la fermeture à glissière (175) le long de l'axe longitudinal (161) ; et
un harnais de tubulures fonctionnellement accouplé au vêtement pour le bras (160) au niveau d'une région de coude du vêtement pour le bras (160).
